# EUROPÄISCHE PATENTANMELDUNG

(11) **EP 2 545 943 A1**
(43) Veröffentlichungstag der Anmeldung: **16.01.2013**
(21) Anmeldenummer: 11173663.3
(22) Anmeldetag: 12.07.2011
(51) Int. Cl.: A61L 15/28, A61L 15/32, A61L 15/42, A61L 15/58

(54) **Perforiertes, geschichtetes Wundbehandlungsmaterial**

(71) Anmelder: Dr. Suwelack Skin & Health Care AG, 48727 Billerbeck (DE)
(72) Erfinder: Wieland, Martin, Dr., 48653 Coesfeld (DE); Grill, Antje, 48151 Münster (DE)
(74) Vertreter: Gille Hrabal

(57) **Zusammenfassung**

Die vorliegende Erfindung betrifft Wundbehandlungsmaterialien mit mindestens zwei perforierten Biomatrixschichten die mittels eines Verbindungsmittels miteinander verbunden sind, sowie Verfahren zur Herstellung derartiger Wundbehandlungsmaterialien und deren Verwendung insbesondere in der Behandlung exsudierender Wunden sowie in der Vakuum-unterstützen Wundbehandlungstherapie.

## Beschreibung

Die vorliegende Erfindung betrifft Wundbehandlungsmaterialien mit mindestens zwei perforierten Biomatrixschichten die mittels eines Verbindungsmittels miteinander verbunden sind, sowie Verfahren zur Herstellung derartiger Wundbehandlungsmaterialien und deren Verwendung insbesondere in der Behandlung exsudierender Wunden sowie in der Vakuum-unterstützen Wundbehandlungstherapie.

### EINLEITUNG UND STAND DER TECHNIK

Im Bereich der Wundversorgung oder Wundbehandlung hat sich neben der etablierten Verwendung von Wundauflagen außerdem die Anwendung von mechanischen und elektrischen Behandlungsverfahren durchgesetzt. Insbesondere hat sich das Unterdruck- oder Vakuum-Verfahren etabliert, um eine schnellere Wundheilung zu erreichen. Unter dem Begriff Negative Pressure Wound Therapy (NPWT) oder Vakuumtherapie ist dieses Verfahren im medizinischen Umfeld bekannt. Bei dieser Therapieform wird eine, zumeist tiefe Wunde mit einem synthetischen oder semi-synthetischen Füll-, Schwamm- oder Schaummaterial, beispielsweise einer Baumwollgaze oder einem Polyurethan- (PU-), Polyethylen- (PE-) oder Polyvinylalkohol- (PVA-) Schwamm ausstaffiert und mit einer dünnen Polymerfolie luftdicht abgeklebt. In diesen Wundbereich wird dann über einen Schlauch mittels einer externen Pumpe ein Unterdruck, der in der Regel bei 80 - 140mm unter Außendruck liegt, angelegt. Durch diesen Effekt werden die Wundränder zusammengezogen und Wundflüssigkeit abgeleitet. Ferner können durch "Mikromassageeffekte" (auch als "Microstrain"-Effekt bezeichnet) die Zellen zum Wachstum und zur Gewebeneubildung angeregt werden.

Aufgrund der Heilungserfolge, die mit dieser Methode erzielt werden können, wird dieses Verfahren weltweit erfolgreich angewandt.

Im Verlauf der Wundheilung wird durch neues Zellwachstum frisches, gesundes Gewebe gebildet. Eine verbesserte Gewebeneubildung wird dabei insbesondere bei der Anwendung einer Vakuum-unterstützten Wundbehandlung beobachtet. Dabei zeigen Gewebezellen allerdings die Tendenz in die verwendeten Füll- oder Schwamm-Materialien einzuwachsen.

Entsprechend haben sich bei der herkömmlichen Anwendung insbesondere folgende Nachteile gezeigt:

Bei der Verwendung von synthetischen Füll-, Schwamm- oder SchaumMaterialien insbesondere bei den üblicherweise verwendeten Polyurethanschäumen und anderen herkömmlichen synthetischen und semi-synthetischen Wundauflagen kommt es zu einem Verwachsen des neu gebildeten Gewebes mit dem Füllmaterial bzw. Schwamm oder Schaum. Dies führt dazu, dass das Füllmaterial beim Verbandswechsel bzw. nach Abschluss der Therapie nicht einfach aus der Wunde entnommen werden kann, sondern häufig chirurgisch entfernt werden muss. Dadurch kann eine Schädigung des neu gebildeten einheilenden Gewebes erfolgen, im schlimmsten Fall wird das neu gebildete Gewebe durch den Eingriff wieder entfernt was beides grundsätzlich abträglich für den Verlauf der Wundheilung ist. Darüber hinaus stellt ein derartiger zusätzlicher chirurgischer Eingriff einen erheblichen Mehraufwand dar, der mit unnötigen Schmerzen beim Patienten und einer Therapieverzögerung verbunden ist.

Darüber hinaus besitzt das unter einer herkömmliche Vakuum-Therapie neu gebildete Gewebe häufig keine hohe Belastbarkeit, ist kosmetisch minderwertig und die verheilte Stelle neigt daher dazu, sich wieder zu öffnen bzw. muss schonend behandelt werden. Dies stellt eine nicht unerhebliche Beeinträchtigung der Lebensqualität der Patienten dar.

Um insbesondere das Problem der Durchwachsung des Füllmaterials zu überkommen existieren zahlreiche Ansätze, worin in der Vakuumtherapie wundseitig sogenannte biodegradierbare oder bioresorbierbare Materialien wie beispielsweise Biomatrixmaterialien bzw. Materialien natürlichen Ursprungs eingesetzt werden. Entsprechend sind Wundbehandlungsmaterialien auf Basis von Biomatrixmaterialien zur Behandlung chronischer und exsudierender Wunden sowie insbesondere auch zur Verwendung in der Vakuum-unterstützten Wundbehandlungstherapie prinzipiell bereits bekannt.

So beschreibt beispielsweise die WO 2007/016664 ein Verfahren zur Wundbehandlung mittels Vakuumtherapie, worin in die Wunde ein granuläres bzw. partikuläres Collagenmaterial oder eine dispergierte Collagen-Masse eingebracht, mit herkömmlichen Wundverbandmaterialien abgedeckt und an ein Vakuum-Drainagesystem angeschlossen wird, um eine bessere Verträglichkeit der Vakuum-Therapie zu erzielen. Die Verwendung von Collagen in granulärer oder partikulärer Form bzw. einer dispersen Masse ist insofern unvorteilhaft, als es sich zwar einerseits gut in tiefe Wunden oder Hautdefekte einstreuen oder einbringen lässt, dort allerdings nach Kontakt mit der Wundflüssigkeit nur schwer bis gar nicht in der Positionierung korrigiert werden dann, da es meist sofort mit dem Wundgrund verklebt oder daran adhäriert. Eine gleichmäßige, flächige Aufbringung ist insbesondere bei der Verwendung von granulärem oder partikulärem Material nur schwer möglich und hängt in der Regel stark vom Geschick und der Erfahrung des Anwenders ab.

Ähnliche Probleme zeigen sich bei der Verwendung von Materialien in Form dispergierter Massen, die sich zwar gut an den Wundoberflächen anmodellieren lassen, jedoch nur bedingt homogen auf die Wundflächen aufgebracht werden können. Darüber hinaus besteht bei derartigen Wasser-basierten Zubereitungen immer das Problem der Stabilisierung und Konservierung gegen mikrobiellen Verderb. Dies bedingt einerseits besondere zusätzliche Aufwendungen bei der Herstellung solcher Produkte und birgt außerdem insbesondere bei Verwendung chemischer Konservierungsstoffe das Risiko unerwünschter Nebenwirkungen oder Unverträglichkeitsreaktionen bei der Anwendung.

Weitere Ansätze umfassen die Verwendung schichtförmiger Biomaterialien, insbesondere solcher in Form eines Sheets, Vlieses, Pads, Layers oder in der Art einer Maske oder Kompresse, die auf mindestens einen Teilbereich des menschlichen oder tierischen Körpers aufgebracht werden können und die aufgrund ihrer Bioresorbierbarkeit im Körper verbleiben und ggf. physiologisch umgesetzt oder abgebaut werden können oder im Rahmen der Behandlung wieder entfernt werden.

Wundbehandlungsmaterialien mit einer solchen schichtförmigen Biomatrix auf Basis von Collagen und deren Verwendung in der Vakuumtherapie sind beispielsweise Gegenstand der US 2003/0078532 sowie der US 2007/0027414. Dabei betreffen beide Druckschriften jedoch ausschließlich solche Materialien worin eine einzige bioresorbierbare Collagen-Schicht in Verbindung mit einer bioinerten synthetischen Füll- oder Deckschicht, wie einem synthetischen Schwamm-Material, vorliegt. Die bioinerten synthetischen Materialien können Perforationen aufweisen. Eine perforierte Biomatrixschicht oder ein Material welches mehrere miteinander verbundene Biomatrixschichten aufweist, wird hierin nicht erwähnt.

Auch die US 2002/115952 beschreibt ein Wundbehandlungsmaterial für die Vakuum-Therapie, worin eine wundseitige bioabsorbierbare Polymermatrix, beispielsweise aus Collagen, mit einem synthetischen offen-zelligen Polyurethan- oder Polyethylenschaum verbunden ist. Darin wird außerdem die Möglichkeit beschrieben, bei einem Verbandswechsel das synthetische Schaummaterial zu entfernen und die bioabsorbierbare Schicht in der Wunde zu belassen. Auch hierin wird ein Material, welches mehrere miteinander verbundene Biomatrixschichten aufweist, nicht erwähnt. Ferner wird auch eine Perforation der bioresorbierbaren Schicht nicht beschrieben.

Des weiteren betrifft beispielsweise die WO 2009/012438 ein Vakuum-Wundbehandlungsmaterial in Form eines porösen Gerüstmaterials, welches mit einem blutstillenden Mittel wie beispielsweise Collagen beschichtet ist. Eine derartige Beschichtung liegt im Bereich 0,01-100 µm.

Gegenstand der WO 2005/123170 sind außerdem Vakuum-Wundbehandlungsmaterialien auf Basis biodegradierbarer Gerüstmaterialien, insbesondere aus Mischungen von Collagen und oxidierter regenerierter Cellulose, die in Verbindung mit nicht-resorbierbaren Materialien vorliegen können.

Beide vorstehenden Dokumente beschreiben keine Materialien die mehrere miteinander verbundene perforierte Biomatrixschichten aufweisen.

Die US 2007/0185426 hat ebenfalls Vakuum-Wundbehandlungsmaterialien zum Gegenstand, wobei hierin insbesondere 3-schichtige Materialien beschrieben werden umfassend wundseitig eine bioresorbierbare Collagenschicht, eine mittlere Schicht eines Hydrogel-bildenden bzw. wasserlöslichen Polymers wie z.B. aus Gelatine, Collagen, Alginat oder Chitosan, sowie eine Abschluss-Schicht eines synthetischen offen-zelligen Polyurethan- oder Polyethylenschaums. Dabei ermöglicht die wasserlösliche Hydrogelschicht einerseits die Ablösung des synthetischen Schaums von der bioresorbierbaren Collagenschicht, andererseits fungiert diese Hydrogelschicht als Barriereschicht, die ein Einwachsen der Zellen in den darüberliegenden PU-Schaum verhindert. Dadurch wird allerdings die Wundheilung und das Zellwachstum mit Erreichen der Barriereschicht gezielt gestoppt. Somit werden auch hierin keine Materialien beschrieben, die mehrere miteinander, beispielsweise über derartige Hydrogelschichten verbundene Biomatrixschichten aufweisen und die außerdem eine schichtweise Durchsiedelung der einzelnen Schichten mit Zellen ermöglichen. Auch eine Perforation der Collagenschicht wird nicht erwähnt.

Ein weiteres Vakuum-Wundbehandlungsmaterial mit einer Biomatrixschicht, insbesondere einer Collagenschicht, ist Gegenstand der US 2001/0043943 bzw. der korrespondierenden US 2003/0208149 und WO 2001/089431. Bei dem darin verwendeten Collagen-Material handelt es sich um gereinigtes, azellularisiertes Dünndarm-Submucosa Gewebe ("small intestine submucosa"; SIS) und somit um intaktes gereinigtes Gewebematerial, dass auch mit Perforationen versehen werden kann. Dabei wird das Collagen-Material mit einer weiteren Deck- oder Füllschicht zusammen in die Wunde eingebracht. Es wird zwar erwähnt, dass auch mehrere Schichten SIS-Material verwendet werden können, allerdings wird nicht erwähnt, diese mittels eines Verbindungsmittels, insbesondere eines wasserlöslichen Verbindungsmittels, miteinander zu verbinden. Auch werden hierin insbesondere keine porösen gerüstartigen Collagen-Materialien zur Bildung der miteinander verbundenen perforierten Biomatrixschichten erwähnt, sondern ausschließlich intaktes Gewebematerial (SIS).

### AUFGABENSTELLUNG

Die Aufgabe der vorliegenden Erfindung bestand nun darin, ein neues und verbessertes Wundbehandlungsmaterial insbesondere zur Verwendung in der Behandlung exsudierender Wunden sowie zur Verwendung in der Vakuum-Wundbehandlungstherapie bereitzustellen, das über eine hohe Verträglichkeit, optimale Resorbierbarkeit, gute und einfache Applizierbarkeit sowie ausreichende Permeationseigenschaften verfügt und außerdem eine optimale Überwachung des Wundheilungsverlaufs ermöglicht.

### BESCHREIBUNG DER ERFINDUNG

Die Erfinder der vorliegenden Erfindung fanden überraschend, dass die vorliegende Aufgabe gelöst werden konnte durch Bereitstellung von Wundbehandlungsmaterialien mit mindestens zwei perforierten Biomatrixschichten die mittels eines Verbindungsmittels miteinander verbunden sind.

Bei den mittels Vakuum-Therapie zu behandelnden Wunden handelt es sich zumeist um tiefe Wunde, d.h. Wunden die tiefer als 0,5 cm sind. Die eingesetzten Wundbehandlungsmaterialien sollten daher auch eine hinreichende Gesamtschichtdicke besitzen, um die Wunde auszukleiden. Die gebräuchlichen PU-Schäume haben eine Dicke zwischen 1,5 und 3,0 cm und werden unter Unterdruck auf ca. 0,4 bis 0,6 cm komprimiert.

Collagenmaterialien sind in der Regel weniger stark komprimierbar und werden aufgrund ihrer physiko-chemischen Struktur sehr schnell besiedelt, so dass hier prinzipiell dünnere Auflagen mit ca. 0,4 bis 2,0 cm Gesamtschichtdicke ausreichend wären. Durch die geringere Komprimierbarkeit lässt sich ein derartiges Collagenmaterial allerdings nicht so gut an Wundunebenheiten anpassen wie die herkömmlichen synthetischen PU-/ oder PE-Schwämme.

Bei der Verwendung von Wundbehandlungsmaterialien in der Vakuum-Therapie muss ferner darauf geachtet werden, dass die Wundbehandlungsmaterialien einen guten Abfluss von Wundexsudat gewährleisten, also eine ausreichende Permeationskapazität aufweisen. Beanspruchte Wunden, z.B. chronische Wunden, erzeugen eine große Menge von Wundflüssigkeit, die die Wundheilung stören kann, da eine übermäßige Exsudation zu Ödembildung und Mazeration führen kann. Derartige Wunden bezeichnet man auch als exsudierende oder stark exsudierende Wunden. Durch das Anlegen von Unterdruck ist es möglich, die Wundflüssigkeit (Exsudat) abzuführen und den Unterdruck durch die Wundauflage auf den Wundgrund zu übertragen.

Bei der Verwendung von herkömmlichen schichtförmigen Collagenmaterialien zeigte sich in Laborversuchen an einer simulierten Wundoberfläche, dass es schnell zu einem Abfallen des Unterdruckes sowie zu einem schlechten Exsudatabfluss kam, da das Collagen mit dem Exsudat wechselwirkte und verstopfte und in seiner Permeationskapazität so weit herabgesetzt wurde, dass kein wirksames Vakuum angelegt werden konnte.

Wird vor diesem Hintergrund lediglich eine dünne Collagenschicht in Kombination mit den herkömmlichen Vakuum-geeigneten synthetischen Schaummaterialien eingesetzt, wie aus dem Stand der Technik bekannt, so besteht insbesondere bei langen Verbandswechselintervallen oder bei schnell granulierenden Wunden die Gefahr, dass die einzelne dünne Collagenschicht schnell vollständig durchwachsen und anschließend auch die darüber liegende synthetische Schaumschicht von Zellen besiedelt wird, womit sich wiederum die Notwendigkeit der chirurgischen Entfernung ergeben kann.

Dazu ist insbesondere auch zu berücksichtigen, dass Wunden unterschiedlich schnell heilen können. Es kann daher von Fall zu Fall jeweils nur bedingt abgeschätzt werden wie schnell der Wundgrund in die bioresorbierbare Matrixschicht einheilt bzw. vordringt oder ob bereits ein Einwachsen in die darüberliegenden synthetischen Füllschichten erfolgt ist. Bei einem Wechsel der Wundauflage ist es daher für den behandelnden Anwender schwer abzuschätzen, wie gut der Heilungsverlauf vonstattengegangen ist, da sich die zuwachsende Wunde quasi inmitten des Wundbehandlungsmaterials befindet. Um den Heilungsfortschritt festzustellen, müsste diese gewaltsam geöffnet oder zerschnitten werden.

Als Extremfall ist es denkbar, dass die Wunde sehr schnell zuwächst und die Wundauflage bereits durchwachsen hat, bevor ein Verbandswechsel stattfindet. In diesem Fall droht der Wundgrund mit der abdeckenden synthetischen Füllschicht, in der Regel ein synthetisches Füll- oder Schaummaterial, zu verwachsen oder zu verkleben, was aus den dargestellten Gründen zu Schwierigkeiten beim Verbandswechsel oder bei der Heilung führen kann.

Vor diesem Hintergrund fanden die Erfinder der vorliegenden Erfindung überraschend, dass ein Wundbehandlungsmaterial, das aus mehreren miteinander verbunden Biomatrixschichten aufgebaut ist, eine besonders geeignete Alternative zu den bekannten Schichtmaterialien aus einer Biomatrixschicht auf einem synthetischen Füllmaterial darstellt.

Durch geeignete Wahl der Anzahl der Biomatrixschichten sowie durch Variation der Schichtdicken der einzelnen Biomatrixschichten kann die Gesamtschichtdicke des Wundbehandlungsmaterials beliebig variiert und an die erforderlichen Behandlungsbedingungen optimal angepasst werden.

Durch Verwendung eines geschichteten Materials wird dem Anwender außerdem ermöglicht, bei einem Verbandswechsel das Material schichtweise abzutragen bzw. zu entfernen und beispielsweise zunächst nur soviele Schichten der Wundauflage abzunehmen, bis er zu den mit Gewebe durchwachsenen Schichten, dem sogenannten Wundgrund, kommt. Wie bereits erwähnt, können aufgrund der unterschiedlichen Heilungsgeschwindigkeit eine oder mehrere der wundseitigen Lagen durchwachsen sein. Die oberen, noch nicht durchwachsenen Schichten der Wundauflage können somit leicht entfernt werden, ohne dass eine Traumatisierung des Wundgrundes erfolgt.

Als besonders vorteilhaft haben sich Verbindungsmittel erwiesen, die in wässriger Umgebung, insbesondere unter physiologischen Wundbedingungen bzw. in Wundexsudat, löslich bzw. trennbar sind und beim Durchfeuchten des Wundbehandlungsmaterials mit dem Exsudat soweit gelöst bzw. getrennt werden, dass die einzelnen Biomatrixschichten leicht voneinander gelöst und abgetrennt werden können, insbesondere ohne Anwendung mechanischer Hilfsmittel, chirurgischer Eingriffe oder hoher Kraftaufwendung.

Um die genannten Probleme der Abnahme der Permeationskapazität und Flüssigkeitsdurchlässigkeit mit zunehmender Wundbehandlungsmaterialdicke zu vermeiden, ist es maßgeblich, dass die miteinander verbundenen Biomatrixschichten Perforationen aufweisen und dass insbesondere gewährleistet ist, dass diese perforierten Biomatrixschichten derart miteinander verbunden sind, dass durch die Gesamtdicke des Wundbehandlungsmaterials durchgehende Perforationen, Öffnungen oder Kanälevorhanden sind oder dass bei Auflösung des Verbindungsmittels derartige durchgehende Perforationen, Öffnungen oder Kanäle gebildet werden, damit die Permeation bzw. der Flüssigkeitsdurchtritt durch die gesamte Schichtdicke des zusammengesetzten Materials während des gesamten Behandlungsverlaufs gewährleistet ist.

Die Wundauflage wird beim Einsatz in exsudierenden Wunden in der Regel innerhalb weniger Stunden komplett befeuchtet und das Material sollte prinzipiell für den Einsatz über mehrere Tage, vorzugsweise über 2 bis 7 Tage geeignet sein. Ein Anwendungszeitraum bis zu 14 Tage ist prinzipiell möglich.

Insbesondere bei Verwendung von Verbindungsmitteln, die in physiologischem Wundmilieu löslich sind, zeigt sich, dass mit dem Auflösen bzw. der Herabsetzung der Haftung bzw. dem Lösen der Verbindung der einzelnen Biomatrixschichten diese gegeneinander verschiebbar werden, quasi im Wundmilieu aufeinander "schwimmen". Dadurch können die Löcher, Schlitze bzw. Öffnungen der einzelnen perforierten Biomatrixschichten, die ursprünglich im fest verbundenen Zustand direkt übereinander angeordnet sind, sich derart gegeneinander verschieben, dass Öffnungen einzelner Schichten vollständig von darüber- oder darunterliegenden Schichten verdeckt werden und damit keine durchgängigen Perforationen durch die Gesamtdicke des Wundbehandlungsmaterials mehr vorliegen, womit der Abfluss verschlechtert und die Permeationskapazität herabgesetzt wird. Im Extremfall können sich die Lagen soweit verschieben, dass der Abfluss komplett blockiert ist.

Um diesem nachteiligen und unerwünschten Verschluß der durchgehenden Perforationen entgegenzuwirken, werden die einzelnen perforierten Biomatrixschichten bevorzugt derart angeordnet, dass die Löcher, Schlitze bzw. Öffnungen der Perforationen der einzelnen Lagen auch bei einer Verschiebung der einzelnen Lagen einen durchgängigen Kanal bilden und damit jederzeit ein Abfluss von Wundexsudat gewährleistet ist. Dies kann durch Anbringen geeigneter Perforationsmuster sowie durch geeignete Kombination und Variation der Perforationsmuster der einzelnen Schichten erreicht werden. Beispielsweise kann dies durch das Einfügen zusätzlicher Löcher oder Schlitze in jeder zweiten Lage bzw. dem geschickten Anordnen von Schlitzen, z.B. durch kreuzförmige Anordnung zweier Lagen übereinander erreicht werden.

Überraschenderweise weisen die so erhaltene Wundbehandlungsmaterialien außerdem eine höhere Flexibilität und somit eine bessere Anpassungsfähigkeit an die Wunde gegenüber einstückigen (nicht-geschichteten) Biomatrixmaterialien vergleichbarer Gesamtschichtdicke auf.

Als weiterer Vorteil der Perforationen zeigte sich, dass es durch die Löcher oder Schlitze einfacher ist, seitlich mit der Wunde verwachsene Lagen wieder abzulösen, da die löchrige Struktur eine Art Perforation vorgibt, an der durch Schneiden oder Reißen nicht eingewachsene Schichtreste abgetrennt werden können, so dass randseitig eingewachsene Teile einer Biomatrixschicht in der Wunde verbleiben können, während der zentrale bzw. innenliegende Hauptanteil dieser Lage entfernt wird.

Das erfindungsgemäße Wundbehandungsmaterial wird maßgeblich aus zwei oder mehr Biomatrixschichten gebildet, welche für die erfindungsgemäße Anwendung als Haut- bzw. Wundauflage, ggf. auch zur Applikation von Wirk- und Pflegesubstanzen, sowie insbesondere zur Aufnahme von Flüssigkeiten wie Wundexsudat oder Blut geeignet sind.

Dabei bezeichnet eine Biomatrixschicht im Sinne der vorliegenden Erfindung eine schichtförmige Biomatrix bzw. eine aus einem biokompatiblen im wesentlichen vollständig oder teilweise bioresorbierbaren oder bioabsorbierbaren Trägermaterial gebildete Auflage, die in Form einer Schicht, eines Sheets, Vlieses, Pads, Layers, Schwamms oder Schaums oder in der Art einer Maske oder Kompresse u.ä. ausgestaltet ist und die eine im wesentlichen flächige Ausgestaltung aufweist.

Die Trägermaterialien der erfindungsgemäßen Biomatrixschichten weisen eine gute Biokompatibilität auf, sind somit haut- und schleimhautverträglich und weisen weder bei der Anwendung auf intakter Haut noch beim Einbringen in Wunden ein toxikologisches Potential auf oder rufen Irritationswirkungen oder andere Unverträglichkeitsreaktionen hervor und sind pharmakologisch unbedenklich.

Dabei werden die erfindungsgemäßen Wundbehandlungsmaterialien aus mindestens zwei solcher schichtförmiger Biomatrix-Lagen in der Art eines sogenannten "sandwich-layers" zusammengesetzt. Erfindungsgemäß bezeichnet eine Biomatrixschicht insbesondere solche mit einer Schichtdicke (kürzeste Seitenlänge) von maximal 10 mm.

Bekannte und gebräuchliche schichtförmige Biomatrices in Form flächiger Ausgestaltungen z.B. in Form von Sheets, Vliesen, Kompressen, Pads u.ä. umfassen beispielsweise flächige oder schichtförmige durchgängige Wundauflagen auf Basis von Collagen aus dem Bereich der Behandlung von chronischen Wunden oder der Hämostyptika, wie solche die unter der Bezeichnung Matriderm®, Matristypt® oder Puracol® von der Firma Dr. Suwelack Skin & Health Care AG oder als Suprasorb C® von der Firma Lohmann & Rauscher, als Promogran® von der Firma Johnson & Johnson bzw. Systagenics oder als Avitene® Sheets von Davol Inc. bekannt sind. Weitere Wundbehandlungsmittel, die auch im Bereich Vakuum-Therapie eingesetzt werden, sind beispielsweise unter der Bezeichnung Integra single layer® oder Integra bilayer® von der Firma Integra® im Handel. Desweiteren sind bekannte Beispiele flächiger, schichtförmiger Wundauflagen auf Basis von Polysacchariden u.a. Algisite M® von Smith & Nephew, Askina Sorb® von B. Braun sowie zahlreiche weitere. Auch flächige, sheetförmige Wundauflagen auf Basis von anderen Polysacchariden wie z.B. Chitoskin® von Sangui BioTech GmbH oder Mischungen von z.B. Collagen und Alginat wie z.B. Fibracol® von Johnson & Johnson sind bekannt und werden als gängige Wundbehandlungsmittel sowohl bei chronischen Wunden als auch als Hämostyptika oder blutstillende Mittel eingesetzt.

Prinzipiell können auch solche bekannten Wundauflagen auf Basis von Biomatrix-Materialien in den erfindungsgemäßen Wundbehandlungsmaterialien zur Bildung der perforierten Biomatrixschichten verwendet werden.

Bevorzugt sind insbesondere solche flächig oder schichtförmig ausgestalteten Biomatrix-Materialien, die sich durch eine gute und gleichmäßige großflächige Applizierbarkeit und eine gute Modellierbarkeit und Positionierbarkeit auf der zu behandelnden Hautfläche auszeichnen, was insbesondere durch Verwendung von Biomatrixschichten mit hoher Flexibilität für eine räumliche bzw. dreidimensionale Modellierbarkeit gewährleistet wird. Insbesondere bei der Behandlung von Wunden mit tiefen Hautdefekten oder großen Wundhöhlen kann dadurch eine möglichst vollständige und homogene Ausfüllung bzw. Auskleidung oder Tamponade solcher tieferen Hautdefekte oder Wundhöhlen mit dem flexiblen Biomatrixmaterial erreicht werden.

Weiterhin muss das Trägermaterial so ausgewählt werden, dass dieses eine ausreichende Stabilität aufweist, um beispielsweise durch Zuschneiden und Perforieren in die erfindungsgemäßen perforierten Biomatrixschichten überführt werden zu können. Darüber hinaus sollten die erfindungsgemäßen Biomatrixschichten eine ausreichende mechanische Stabilität besitzen, um auch während der Verwendung bzw. im Verlauf der Applikation jeweils sowohl im trockenen als auch im angefeuchteten Zustand, formstabil zu bleiben und insbesondere nicht zu reißen.

Erfindungsgemäß wird das Trägermaterial, aus dem die Biomatrixschichten maßgeblich gebildet sind, aus der Gruppe der natürlichen hydrophilen, d.h. mit Wasser benetzbaren Materialien gewählt. Bevorzugt ist ein sogenannter Strukturbildner oder ein strukturbildendes Polymer, insbesondere aus der Gruppe der tierischen und pflanzlichen Hydrokolloide, also ein teilweise wasserlösliches oder wasserquellbares natürliches, strukturbildendes Polymer. Insbesondere bevorzugt sind strukturbildende Hydrokolloide aus den Gruppen der Proteine, der Polysaccharide und/oder der Glucosaminoglykane.

Besonders bevorzugt wird das Trägermaterial der Biomatrixschichten aus der Gruppe der Proteine ausgewählt wie z.B. aus der Gruppe der Collagene, beispielsweise lösliches oder unlösliches, fibrilläres, tierisches oder pflanzliches Collagen, oder Gelatine, Elastin (umfassend auch Elastinhydrolysate), Keratin, Fibroin, Albumine, Globuline wie Lactoglobulin, Milchproteine wie Casein. Dabei ist Collagen ganz besonders bevorzugt, ggf. auch in Mischung mit weiteren fibrillären Proteinen oder in Mischung mit Gelatine oder besonders bevorzugt in Mischung mit Elastin.

Bei Trägermaterialien auf Basis von Collagen handelt es sich bevorzugt um solche, die nach aus dem Stand der Technik und z.B. aus der DE 4028622 oder aus der DE 10350654 bekannten Verfahren aufgearbeitet und hergestellt werden. Die erfindungsgemäß bevorzugten Collagenträgermaterialien zeichnen sich insbesondere durch herausragende Hydratationseigenschaften und eine gute Flüssigkeitsaufnahmekapazität bzw. Saugfähigkeit aus, ein Aspekt der insbesondere zur Aufnahme großer Flüssigkeitsmengen z.B. bei stark exsudierenden oder blutenden Wunden vorteilhaft ist, sowie durch deren antiirritative und wundheilungsfördernde Eigenschaften. Aufgrund der strukturellen Ähnlichkeit mit der menschlichen Haut und menschlichem Gewebe werden bevorzugt Collagenarten ausgewählt, die in Haut und Gewebe auftreten, insbesondere Collagen des Typs I, III und V. Dadurch bedingt sich die besonders gute Verträglichkeit und Biokompatibilität sowie Bioresorbierbarkeit von Biomatrixschichten auf Basis derartiger Collagen-Trägermaterialien, da diese bei Verbleib im Körper oder im Verlauf der Wundheilung biologisch abbaubar sind und auf natürliche Weise verstoffwechselt werden.

Das erfindungsgemäß verwendete Collagen-Trägermaterial wird bevorzugt aus Collagenquellen bovinen, equinen und porcinen Ursprungs gewonnen. Das Collagen kann nach üblichen Verfahren aus den üblichen Quellen wie Häuten oder Sehnen gewonnen werden.

Darüber hinaus können auch Collagenmaterialien verwendet werden, welche einer Vernetzungsreaktion unterworfen wurden. In diesem Fall ist eine thermische Vernetzung, die so genannte Dehydrothermalvernetzung, bevorzugt. Ferner ist die Vernetzung mit chemischen Vernetzern möglich. Hierzu zählen insbesondere Aldehyde, wie Glutaraldehyd; Carbodiimide, wie EDC; Isocyanate; Epoxide oder Imidazole, wobei das Epoxid aus der Gruppe der chemischen Vernetzer besonders bevorzugt ist.

Ebenfalls bevorzugte Trägermaterialien, die aus der Gruppe der Polysaccharide ausgewählt werden, schließen beispielsweise Homoglykane oder Heteroglykane, wie zum Beispiel Alginate, besonders Natriumalginat oder Calciumalginat oder Mischungen davon, Carrageen, Pektine, Tragant, Guar-Gummi, Johannisbrotkernmehl, Agar-Agar, Gummi-Arabikum, Xanthan, natürliche und modifizierte Stärken, Dextrane, Dextrin, Maltodextrine, Chitosan, Glucane wie β-1,3-Glucan oder β-1,4-Glucan, Cellulose, oxidierte regenerierte Cellulose (ORC) etc. ein. Besonders bevorzugte Polysaccharide sind Alginate, insbesondere Natrium-Alginate und Calcium-Alginate sowie oxidierte regenerierte Cellulose oder Mischungen davon.

Die Gruppe der Trägermaterialien, die aus der Gruppe der Polysaccharide ausgewählt sind, umfasst ebenfalls solche Materialien, die einer Vernetzung unterworfen wurden. Insbesondere schließen vernetzte Polysaccharide mit Calcium-lonen vernetzte Alginate ein.

Glucosaminoglycane (GAGs / Mucopolysaccharide) schließen beispielsweise ein Hyaluronsäure, Chondroitinsulfat, Dermatansulfat, Keratansulfat, Heparansulfat, Heparin etc.. Besonders bevorzugt ist Hyaluronsäure.

Weiterhin können auch Biomatrixschichten auf Basis von Trägermaterialien verwendet werden, die aus der Gruppe der bioabsorbierbaren synthetischen bzw. modifizierten natürlichen Polymere, umfassend z.B. Polylactide oder Polymilchsäuren (PLA), Polyglycolsäure (PGA), Polycaprolactone (PCL), Polydioxanone (PDO), Polylactid-co-Glycolide (PLGA), Polytrimethylencarbonat etc. ausgewählt sind.

Es können auch Biomatrixschichten verwendet werden, die Mischungen aus mindestens zwei verschiedenen der oben genannten Trägermaterialien enthalten. Dabei sind insbesondere Biomatrixschichten auf Basis von Mischungen aus Collagen mit Gelatine, Elastin, Alginaten, Glucosaminoglycanen wie Hyaluronsäure (GAGs), PLA oder PGA , sowie auf Basis von Mischungen aus Alginaten mit Collagen, Gelatine, Elastin, Glucosaminoglycanen wie Hyaluronsäure, PLA oder PGA bevorzugt. Gegebenenfalls können solche bevorzugten Mischungen auch weitere der genannten Trägermaterialien enthalten.

Grundsätzlich können die Trägermaterialien der erfindungsgemäßen Biomatrixschichten auch geringe Mengen synthetischer -und/oder semi-synthetischer und/oder modifizierter natürlicher Polymere enthalten, wie z.B. solche die aus der Gruppe umfassend beispielsweise Celluloseether, Polyvinylalkohol, Polyvinylpyrrolidon, synthetische Cellulosederivate, wie Methylcellulose, Carboxycellulose, Carboxymethylcellulose wie z.B. Natrium-Carboxymethylcellulose, Celluloseester, Celluloseether wie Hydroxypropylcellulose, kationisierte Cellulosen oder kationisierte Stärken etc., Polyacrylsäure, Polymethacrylsäure, Poly(methylmethacrylat) (PMMA), Polymethacrylat (PMA), Polyethylenglykole, Polyurethane, Polyharnstoffverbindungen etc. und Mischungen davon ausgewählt sind. Ein bevorzugtes synthetisches Polymer ist Polyacrylat bzw. Polyacrylsäure, welches ganz besonders bevorzugt z.B. in Mischung mit Trägermaterialien die aus den Alginaten ausgewählt sind, in den Biomatrixschichten enthalten sein kann. Bevorzugte semi-synthetische bzw. modifizierte natürliche Polymere sind solche umfassend Cellulose, Carboxymethylcellulose, kationisierte Cellulosen oder kationisierte Stärken.

Der Anteil solcher synthetischer und/oder semi-synthetischer und/oder modifizierter natürlicher Polymere in den erfindungsgemäßen Biomatrixschichten liegt dabei in der Regel unter 40 Gew.-%, bevorzugter unter 25 Gew.-%, noch bevorzugter unter 10 Gew.-%, jeweils bezogen auf das Gesamtgewicht der trockenen Biomatrixschichten.

Ganz besonders bevorzugt sind jedoch solche Biomatrixschichten, die keine synthetischen Trägermaterialien enthalten, wobei hier nicht die vorstehend beschriebenen bioabsorbierbaren synthetischen bzw. modifizierten natürlichen polymeren Trägermaterialien gemeint sind.

Der Anteil der vorstehend genannten bioabsorbierbaren synthetischen bzw. modifizierten natürlichen Polymere in den erfindungsgemäßen Biomatrixschichten liegt in der Regel unter 70 Gew.-%, bevorzugter unter 60 Gew.-%, noch bevorzugter unter 50 Gew.-%, jeweils bezogen auf das Gesamtgewicht der trockenen Biomatrixschichten.

Erfindungsgemäß sind auch Biomatrixschichten auf Basis vernetzter Trägermaterialien geeignet, da vernetzte Materialien über eine besonders hohe mechanische Stabilität verfügen. Dadurch sind vernetzte Trägermaterialien umfassende Biomatrixschichten besonders gut geeignet, um die erfindungsgemäßen Perforationen anzubringen. Besonders bevorzugt sind dabei Biomatrixschichten auf Basis vernetzter Collagene, insbesondere auf Basis dehydrothermal- oder Epoxid-vernetzter Collagene wie sie beispielsweise in der unveröffentlichen EP-Anmeidung 10196934.3 der Anmelderin beschrieben sind, sowie Calcium-vernetzte Alginatmaterialien.

Um eine optimale Granulation bzw. Wundheilung zu erzielen ist es außerdem maßgeblich, dass solche Biomatrixmaterialien gewählt werden, die das Wachstum körpereigener Zellen und damit die Neubildung von intakten Gewebestrukturen unterstützen, was insbesondere bei Vorliegen einer Gerüst- oder sogenannten "scaffold"-Funktion gegeben ist. Überraschenderweise hat sich gezeigt, daß insbesondere gefriergetrocknete, poröse Biomatrixmaterialien trotz ihrer vergleichsweise feinen Porosität ihrer schwammartigen Struktur über eine hohe Biokompatibilität und Besiedelbarkeit verfügen. Aus dem Stand der Technik sind auch fein-porige synthetische Materialien bekannt, wobei diese in der Regel keine Besiedelbarkeit ermöglichen. Die besondere Eignung der feinporigen gefriergetrockneten Collagenmaterialien ist dabei neben deren "scaffold"-Funktion insbesondere auch auf deren strukturelle Ähnlichkeit mit Haut und Gewebe, sowie auf die durch das Herstellverfahren bedingte hohe Homogenität zurückzuführen.

Die erfindungsgemäßen Biomatrixschichten sind bevorzugt erhältlich durch ein Verfahren, welches die Schritte umfasst:
a) Herstellen einer wässrigen Suspension oder einer Lösung mindestens eines strukturbildenden natürlichen Polymers;
b) gegebenenfalls Einmischen eines oder mehrerer Wirk- und/oder Hilfsstoffe;
c) Gießen der Mischung in eine geeignete Form;
d) Trocknen, vorzugsweise Gefriertrocknen, der Mischung unter Erhalt eines porösen Formkörpers;
e) gegebenenfalls Schneiden des aus Schritt d) erhältlichen Formkörpers in Schichten;
f) gegebenenfalls Schneiden der Schichten aus Schritt e) in die gewünschte geometrische Ausgestaltung der Biomatrixschicht und
g) gegebenenfalls Perforieren der Biomatrixschicht.

Bevorzugt werden in Schritt c) großformatige Platten oder Blöcke mit vergleichsweise großer Dicke (ca. 1 bis 10 cm) gegossen, die dann in Schritt e) in die gewünschte Schichtdicke von 0,5 bis 10 mm geschnitten werden. Es ist jedoch auch möglich, die Materialien im Schritt c) unmittelbar in der gewünschten Schichtdicke zu Gießen und zu Trocknen.

Geeignete Verfahren zum Erhalt derartiger gefriergetrockneter poröser Biomatrices sind beispielsweise in der DE 4028622, DE 10350654, der WO 04/104076, der WO 05/113656 oder der WO 08/020066 der Anmelderin beschrieben, welche damit vollumfänglich im Offenbarungsgehalt umfasst sind.

Gemäß der vorstehenden Ausführungen können die einzelnen Biomatrixschichten entweder vollständig aus einem geeigneten Trägermaterial gebildet sein oder zu einem überwiegenden Teil daraus bestehen. Beispielsweise können die Biomatrixschichten auf einem Trägermaterial basieren, dem zusätzliche Wirk- und/oder Hilfsstoffe zugesetzt wurden.

Wirkstoffe schließen insbesondere kosmetische oder therapeutische bzw. pharmazeutische, für die äußere Anwendung geeignete Wirkstoffe ein.

Beispiele kosmetisch gegebenenfalls auch z.B. dermatologischer, therapeutisch wirksamer Verbindungen schließen ein: antimikrobielle Mittel wie Antiseptika und Antibiotika, adstringierende Mittel, Konditionierungsmittel für die Haut, hautglättende Mittel, Mittel zur Steigerung der Hauthydratation wie z.B. Glycerin oder Harnstoff, Keratolytika, Radikalfänger für freie Radikale, Wirkstoffe, die die Differenzierung und/oder Proliferation und/oder Pigmentierung der Haut modulieren, Vitamine und Provitamine wie z.B. Beta-Carotin, Vitamin A, B, E etc., Vitamin C (Ascorbinsäure) und ihre Derivate, wie beispielsweise Glycoside wie Ascorbylglucosid, oder Ester der Ascorbinsäure wie Natrium- oder Magnesium-Ascorbylphosphat oder Ascorbylpalmitat und -stearat L-Ascorbinsäurephosphatester, Alkalimetallsalze wie Natrium- und Kaliumsalze von L-Ascorbinsäurephosphatestern; Erdalkalimetallsalze wie Magnesium- und Calciumsalze von L-Ascorbinsäurephosphatestern; trivalente Metallsalze wie Aluminiumsalze von L-Ascorbinsäurephosphatestern; Alkalimetallsalze von L-Ascorbinsäuresulfatestern wie Natrium- und Kaliumsalze von L-Ascorbinsäuresulfatestern; Erdalkalimetallsalze wie Magnesium- und Calciumsalze von L-Ascorbinsäuresulfatestern; trivalente Metallsalze wie Aluminiumsalze von L-Ascorbinsäuresulfatestern; Alkalimetallsalze wie Natrium- und Kaliumsalze von L-Ascorbinsäureestern; Erdalkalimetallsalze wie Magnesium- und Calciumsalze von L-Ascorbinsäureestern; und trivalente Metallsalze wie Aluminiumsalze von L-Ascorbinsäureestern.

Wirkstoffe mit reizender Nebenwirkung, wie alpha-Hydroxysäuren, β-Hydroxysäuren, alpha-Ketosäuren, β-Ketosäuren, Retinoide (Retinol, Retinal, Retin-Säure) Anthralinen (Dioxyanthranol), Anthranoide, Peroxide (insbesondere Benzoylperoxid), Minoxidil, Lithiumsalze, Antimetabolite, Vitamin D und seine Derivate; Katechine, Flavonoide, Ceramide, mehrfach ungesättigte Fettsäuren, essentielle Fettsäuren (z.B. gamma-Linolensäure), Enzyme, Coenzyme, Enzymhemmstoffe, hydratisierende Mittel, hautberuhigende Mittel, Detergentien oder schaumbildende Mittel, und anorganische oder synthetische mattierende Füllstoffe, oder dekorative Stoffe wie Pigmente oder Farbstoffe und -partikel.

Weiterhin können Pflanzenwirkstoffextrakte bzw. daraus gewonnene Auszüge oder Einzelstoffe erwähnt werden. Allgemein wird der Pflanzenwirkstoffextrakt in der Regel ausgewählt aus der Gruppe bestehend aus festen Pflanzenextrakten, flüssigen Pflanzenextrakten, hydrophilen Pflanzenextrakten, lipophilen Pflanzenextrakten, einzelnen Pflanzeninhaltsstoffen; sowie deren Mischungen, wie Flavonoide und ihre Aglyka: Rutin, Quercetin, Diosmin, Hyperosid, (Neo)hesperidin, Hesperitin, Ginkgo Biloba (z.B. Ginkoflavonglykoside), Crataegus-Extrakt (z.B. oligomere Procyanidine), Buchweizen (z.B. Rutin), Sophora japonica (z.B. Rutin), Birkenblätter (z.B. Quercetinglykoside, Hyperosid und Rutin), Holunderblüten (z.B. Rutin), Lindenblüten (z.B. ätherisches Öl mit Quercetin und Farnesol), Johanniskrautöl, Olivenölauszug, Calendula, Arnika (z.B. ölige Auszüge der Blüten mit ätherischem Öl, polare Auszüge mit Flavonoiden), Melisse (z.B. Flavone, ätherisches Öl); Immunstimulantien: Echinacea purpurea (z.B. alkoholische Auszüge, Frischpflanzensaft, Preßsaft), Eleutherokokkus senticosus; Alkaloide: Koffein, Teein, Theobromin, Rauwolfia (z.B. Prajmalin), Immergrün (z.B. Vincamin); weitere Phytopharmaka: Aloe, Roßkastanie (z.B. Aescin), Knoblauch (z.B. Knoblauchöl), Ananas (z.B. Bromelaine), Ginseng (z.B. Ginsenoside), Mariendistelfrüchte (z.B. auf Silymarin standardisierter Extrakt), Mäusedornwurzel (z.B. Ruscogenin), Baldrian (z.B. Valepotriate, Tct. Valerianae), Kava-Kava (z.B. Kavalactone), Hopfenblüten (z.B. Hopfenbitterstoffe), Extr. Passi-florae, Enzian (z.B. ethanol. Extrakt), anthrachinonhaltige Drogenauszüge, z.B. aloinhaltiger Aloe Vera-Saft, Pollenextrakt, Algenextrakte, Süßholzwurzelextrakte, Palmenextrakt, Galphimia (z.B. Urtinktur), Mistel (z.B. wässrig-ethanol. Auszug), Phytosterole (z.B. beta-Sitosterin), Wollblumen (z.B. wäßrig-alkohol. Extrakt), Drosera (z.B. Likörweinextrakt), Sanddornfrüchte (z.B. daraus gewonnener Saft oder Sanddornöl), Eibischwurzel, Primelwurzelextrakt, Frischpflanzenextrakte aus Malve, Beinwell, Efeu, Schachtelhalm, Schafgarbe, Spitzwegerich (z.B. Preßsaft), Brennessel, Schöllkraut, Petersilie; Pflanzenextrakte aus Norolaena lobata, Tagetes lucida, Teeoma siems, Momordica charantia, Aloe Vera Extrakte, Senföle.

Im Unterschied zu den vorstehend beschriebenen im wesentlichen in der Kosmetik verwendeten Wirkstoffen handelt es sich bei den therapeutischen Wirkstoffen (Arzneimitteln) um solche, die im Sinne des Arzneimittelgesetzes unter anderem dazu bestimmt sind, Krankheiten, Leiden, Körperschäden oder krankhafte Beschwerden zu heilen, zu lindern oder zu verhüten. Erfindungsgemäß sind insbesondere solche Mittel bzw. Wirkstoffe geeignet, die für die äußere oder transdermale Anwendung, insbesondere im Bereich der Wundbehandlung und -heilung sowie im Bereich der Behandlung von Brandverletzungen bestimmt sind.

Bei Wirkstoffen für eine dermale oder transdermale Anwendung handelt es sich insbesondere um hautaktive aber auch um transdermale Wirkstoffe. Sie schließen beispielsweise ein: Mittel zur Behandlung von Brandverletzungen, Mittel zur Behandlung von Hautkrankheiten, äußerlich anwendbare Analgetika, z.B. Dextropropoxyphen, Pentazocin, Pethidin, Buprenorphin; Antirheumatika/Antiphlogistika (NSAR), z.B. Indometacin, Diclofenac, Naproxen, Ketoprofen, Ibuprofen, Flurbiprofen, Salicylsäure und -derivate wie Acetylsalicylsäure, Oxicame; Steroidhormone, z.B. Betamethason, Dexamethason, Methylprednisolon, Ethinylestradiol, Medroergotamin, Dihydroergotoxin; Gichtmittel, z.B. Benzbromaron, Allopurinol; Dermatika Externa, Antihistaminika, Antiseptika wie Silber Sulfadiazin, Chlorhexidin, Povidon Jod, Triclosan, Silbersalze und kolloidales sowie metallisches Silber, Sucralfat, Quarternäre Ammoniumverbindungen etc; Antibiotika wie Tetracyclin, Penicillin, Terramycin, Erythromycin, Bacitracin, Neomycin, Polymycin B, Mupirocin, Clindamycin, Doxicyclin etc.; Antimykotika, Peptidarzneistoffe, antivirale Wirkstoffe, entzündungshemmende Wirkstoffe, juckreizstillende Wirkstoffe, anästhesierende Wirkstoffe, z.B. Benzocain, Corticoide, antiparasitäre Wirkstoffe; äußerlich anwendbare Hormone; Venentherapeutika; Immunsuppresiva etc. alle für die dermale oder transdermale Anwendung; bioaktive Gläser und Silikate mit granulationsfördernder und antiseptischer Wirkung.

Bevorzugte therapeutische Mittel für die dermale und transdermale Anwendung sind Mittel zur Behandlung von Hautkrankheiten, wie insbesondere solche, die im Bereich der Wundbehandlung, insbesondere zur Behandlung von chronischen Wunden, Dekubitus, Ulcus cruris, diabetischem Fußsyndrom etc. eingesetzt werden wie beispielsweise Analgetika, z.B. Immunsuppressiva, Hormone, anästhesierende Wirkstoffe, antiparasitäre, fungizide bzw. antimykotische und antibakterielle Wirkstoffe (Antiseptika und Antibiotika) wie insbesondere Silber-haltige Wirkstoffe wie z.B. Silbernitrat, Silberchlorid, Silberjodid, kolloidales Silber sowie metallisches Silber oder weitere aus dem Stand der Technik bekannte Silber-haltige Wundbehandlungsstoffe, Wirkstoffe zur Unterstützung und Regulierung des Wundmilieus wie insbesondere Elektrolyte, Kieselerde, Mineralstoffe und Spurenelemente wie z.B. Kalium, Magnesium, Calcium, Selen, Iod etc., bioaktive Gläser und Silikate mit granulationsfördernder und antiseptischer Wirkung, Wirkstoffe zur Erzielung eines Wunddebridements wie z.B. Collagenasen oder andere, im Stand der Technik bekannte, geeignete proteolytischen Enzyme sowie Wirkstoffe zur Unterstützung der Wundheilung wie z.B. Wachstumsfaktoren, Enzyminhibitoren, plättchenreiches Plasma, Thrombozyten, sowie extrazelluläre Matrixbestandteile und lösliche (niedermolekulare) Protein- und Peptidbestandteile wie bevorzugt solche aus der Gruppe umfassend Elastin, Elastin-Hydrolysate, Glykosaminoglykane, wie Heparansulfat, Chondroitinsulfat, Dermatansulfat, Keratansulfat, Heparin und Hyaluronsäure, Proteoglykane wie Aggrecan, Fibromodulin, Decorin, Biglycan, Versican, Perlecan, Basalmembran-Proteoglykan hoher Dichte, Syndecan und Serglycin, Fibrin, Fibronectin, Glucane wie Paramylon etc. Ganz besonders bevorzugte derartige extrazelluläre Matrixbestandteile bzw. Strukturbildner sind Elastin und Elastinhydrolysate, Hyaluronsäure und Fibronectin etc..

Weitere bevorzugte Wirkstoffe sind solche, die eine blutstillende oder hämostatische Wirkung aufweisen wie z.B. Thrombin, Fibrinogen oder Cholesterylsulfat (z.B. Natrium-Cholesterylsulfat) oder Wirkstoffe mit aktivierender Wirkung auf Faktoren und Substanzen der extrinsischen und/oder intrinsischen Gerinnungskaskade wie z.B. Phospholipide, Kaolin, Aprotinin, Faktor- oder Faktoren-Konzentrate, Tissue Factor oder Calcium-lonen.

Auch die Trägermaterialien der Biomatrixschichten, insbesondere solche auf Basis proteinogener Polymere wie insbesondere Collagen oder pflanzliche Polymere wie Polysaccharide können gewisse therapeutische Wirkungen aufweisen. So wirkt das bevorzugt verwendete Collagen blutstillend und zeigt einen positiven, unterstützenden Effekt in der Wundheilung. Auch dem bevorzugt verwendeten Hydrokolloid (Natrium-)Alginat wird eine gewisse blutstillende Wirkung nachgesagt. Weiterhin wirkt es in gewissem Ausmaß antiviral. Hyaluronsäure wird eine gewisse Wirkung in der Re-Epithelisierung und als Antioxidans und Feuchtigkeitsspender in der Hautpflege nachgesagt. Sie sind jedoch keine Wirkstoffe im Sinne der Erfindung.

Entsprechend können die perforierten Biomatrixschichten der erfindungsgemäßen Wundbehandlungsmaterialien außerdem mindestens einen Wirkstoff enthalten, wobei bevorzugt mindestens ein Wirkstoff aus der Gruppe der blutstillenden Mittel und/oder aus der Gruppe der Wundbehandlungsmittel enthalten ist.

Die erfindungsgemäßen Biomatrixschichten können ferner mindestens einen Hilfsstoff umfassen.

Hilfsstoffe schließen ein: pH-Einstellungsmittel, wie Pufferstoffe, anorganische und organische Säuren oder Basen; Fettsubstanzen, wie Mineralöle, wie Paraffinöle oder Vaselineöle, Siliconöle, Pflanzenöle wie Kokosöl, Süßmandelöl, Aprikosenöl, Maisöl, Jojobaöl, Olivenöl, Avocadoöl, Sesamöl, Palmöl, Eukalyptusöl, Rosmarinöl, Lavendelöl, Kiefernöl, Thymianöl, Minzöl, Kardamonöl, Orangenblütenöl, Sojaöl, Kleieöl, Reisöl, Rapsöl und Rizinusöl, Weizenkeimöl und daraus isoliertes Vitamin E, Nachtkerzenöl, Pflanzenlecithine (z.B. Sojalecithin), aus Pflanzen isolierte Sphingolipide/Ceramide, tierische Öle oder Fette, wie Talg, Lanolin, Butteröl, Neutralöl, Squalan, Fettsäureester, Ester von Fettalkoholen wie Triglyceride, und Wachse mit einem der Hauttemperatur entsprechenden Schmelzpunkt (tierische Wachse, wie Bienenwachs, Carnaubawachs und Candelillawachs, mineralische Wachse, wie mikrokristalline Wachse, und synthetische Wachse, wie Polyethylen- oder Silikonwachse), sowie sämtliche für kosmetische Zwecke geeigneten Öle (sogenannte kosmetische Öle), wie beispielsweise in der CTFA-Abhandlung, Cosmetic Ingredient Hand-book, 1. Auflg., 1988, The Cosmetic, Toiletry and Fragrance Association, Inc., Washington, erwähnt, oberflächenaktive Mittel neben den oben erwähnten Waschtensiden, wie Dispergiermittel, Netzmittel, Emulgatoren etc.; Füllstoffe; Stabilisatoren; Cosolvenfiien; pharmazeutisch und kosmetisch gebräuchliche oder sonstige Farbstoffe und Pigmente, insbesondere solche, die primär zur farblichen Gestaltung der Biomatrixschichten eingesetzt werden und nicht zur Applikation und farblichen Gestaltung am menschlichen Körper; Konservierungsmittel; Weichmacher; Schmiermittel bzw. Gleitmittel; etc..

Erfindungsgemäß bevorzugte Hilfsstoffe sind Pufferstoffe, pH-Einstellungsmittel, Feuchthaltemittel und Netzmittel.

Generell schließt die Einordnung der vorstehend erwähnten Stoffe in die Kategorie der Hilfsstoffe im Rahmen der vorliegenden Erfindung nicht aus, dass diese Hilfsstoffe auch gewisse kosmetische und/oder therapeutische Wirkungen entfalten können, was in besonderem Maß für die genannten bevorzugt eingesetzten kosmetischen Öle gilt.

Die erfindungsgemäßen Wundbehandlungsmaterialien umfassen mindestens zwei, bevorzugt mindestens drei, noch bevorzugter 3 bis 10 perforierte Biomatrixschichten.

Dabei können die einzelnen Biomatrixschichten der erfindungsgemäßen Wundbehandlungsmaterialien in ihrer Zusammensetzung und/oder in ihrer geometrischen Ausgestaltung bzw. in der Art und Anordnung der jeweils aufgebrachten Perforationen jeweils gleich oder unterschiedlich sein.

Beispielsweise können alle miteinander verbunden Biomatrixschichten aus Collagen gebildet sein. Dabei können bevorzugt im wesentlichen identische Collagenmatrices verwendet werden.

Weitere bevorzugte Ausführungsformen betreffen Wundbehandlungsmaterialien, die in ihrer Zusammensetzung unterschiedliche perforierte Biomatrixschichten aufweisen. Dadurch ist es insbesondere möglich, Wundbehandlungsmaterialien mit einer Art Auflösungs- oder Resorptionsgradienten zur Verfügung zu stellen, in dem Biomatrixschichten mit unterschiedlichem Auflösungs- bzw. Resorptionsverhalten in geeigneter Reihenfolge geschichtet werden. So können beispielsweise wundseitig leichter resorbierbare Biomatrixschichten vorgesehen werden, zum Beispiel solche auf Basis lediglich dehydrothermal vernetzter Collagen-Materialien, auf die weitere Collagen-Matrix Schichten mit zunehmendem Vernetzungsgrad und damit zunehmender Degradationsstabilität bzw. abnehmender Resorbierbarkeit aufgebracht werden.

Auch ist denkbar, Biomatrixschichten auf Basis unterschiedlicher Trägermaterialien miteinander zu kombinieren, beispielsweise durch Kombination von Collagen-Matrixschichten mit weiteren Biomatrixschichten auf Basis von Polysacchariden oder Alginaten. Durch geeignete Auswahl der Biomatrixschicht-Materialien und Schichtungsreihenfolge können somit Wundbehandlungsmaterialien mit variabler und optimal eingestellter Resorbierbarkeit und Applizierbarkeit für ein optimales Besiedelungsergebnis bereitgestellt werden.

Erfindungsgemäß besonders bevorzugt sind Biomatrixschichten auf Basis gefriergetrockneter poröser Biomatrices, insbesondere solche auf Basis von Collagenmatrices, wie sie beispielsweise nach dem vorstehenden genannten Verfahren, bzw. nach den in den vorstehend genannten Druckschriften der Anmelderin beschriebenen Verfahren, erhältlich sind.

Erfindungsgemäß sind insbesondere Wundbehandlungsmaterialien bevorzugt, worin die Biomatrixschichten gefriergetrocknet sind.
Erfindungsgemäß weisen die einzelnen Biomatrixschichten jeweils eine Schichtdicke (definiert als die kürzeste Entfernung zweier Punkte d.h. Schichtdicke) bis zu 10 mm auf. Bevorzugt weisen die einzelnen Biomatrixschichten jeweils eine Schichtdicke bis 6 mm, bevorzugter bis 4 mm, noch bevorzugter bis 3 mm auf. Schichtdicken von 1, 2 und 3 mm sind ganz besonders bevorzugt. Außerdem sind Schichtdicken von mindestens 0,5 mm, bevorzugt, bevorzugter sind mindestens 1 mm dicke Schichten.

Dabei können die einzelnen Biomatrixschichten der erfindungsgemäßen Wundbehandlungsmaterialien hinsichtlich ihrer Schichtdicke ebenfalls jeweils gleich oder verschieden sein. Auch durch Kombination von Biomatrixschichten unterschiedlicher Schichtdicke, neben der Kombination unterschiedlicher Perforationsmuster, zur Bildung der erfindungsgemäßen Wundbehandlungsmaterialien kann das Applikations- und Resorptionsverhalten dieser Materialien gezielt gesteuert werden.

Die aus den perforierten Biomatrixschichten gebildeten erfindungsgemäßen Wundbehandlungsmaterialien weisen bevorzugt eine Gesamtschichtdicke bzw. Gesamtdicke von mindestens 2 mm, bevorzugter mindestens 4 mm auf.

Nach oben hin ist die Gesamtdicke im Prinzip nicht begrenzt, denkbar sind Gesamtdicken bis zu 30 oder gar bis zu 70 mm, wobei Gesamtdicken bis zu 20 mm bevorzugt sind.

Zur Bildung der erfindungsgemäßen Wundbehandlungsmaterialien sind die Biomatrixschichten mittels eines Verbindungsmittels miteinander verbunden.

Verbindungsmittel im Sinne der vorliegenden Erfindung umfassen insbesondere Kleber wie Sprühkleber oder Klebefolien, Fäden und Nahtmaterial, Klammern und andere im chirurgischen Bereich übliche Befestigungsmittel, sowie insbesondere auch in den Biomatrixschichten angebrachte bzw. gebildete Laschen und Schlaufen, mittels denen zwei jeweils benachbarte Biomatrixschichten miteinander verbunden oder verknüpft werden können. Die erfindungsgemäßen Verbindungsmittel müssen insbesondere eine physiologische Verträglichkeit aufweisen.

Bevorzugt werden Kleber verwendet, wie beispielsweise flüssige, halb-feste oder folienförmige Kleber, beispielsweise Polyacrylatkleber (z.B. Poly(butylmethacrylat, methylmethacrylat), Polymethylacrylat, Acrylat-Copolymer, Cyanacrylate; Gewebekleber wie Fibrinkleber, Albumin-/Glutardialdehyd-Kleber; PEG- (Polyethylenglycol-) basierte Kleber; Sprühkleber auf Basis von synthetischen, semi-synthetischen oder natürlichen Filmbildnern (z.B. auf Basis von Nitrocellulose, Hydroxypropylcellulose, Gelatine-, Chitosan- oder Alginat-Lösungen, sowie Mischungen davon), herkömmliche Sprühkleber oder Sprühpflaster etc..

Erfindungsgemäß ist es besonders bevorzugt, dass die Verbindungsmittel in Wasser oder wässrigen Lösungen bzw. in physiologischem Milieu löslich oder resorbierbar sind. Dabei meint löslich oder resorbierbar, dass die Hafteigenschaften bzw. die Bindungskräfte des Verbindungsmittels in Gegenwart von Wasser oder wässrigen Lösungen bzw. in physiologischer Umgebung wie in einer Wunde, insbesondere in Gegenwart von Wundexsudat oder Blut, soweit herabgesetzt werden, dass die miteinander verbundenen Schichten leicht, ohne erhöhten Kraftaufwand oder Einsatz mechanischer Hilfsmittel wie Messer, Skalpell, Spatel etc. voneinander getrennt werden können.

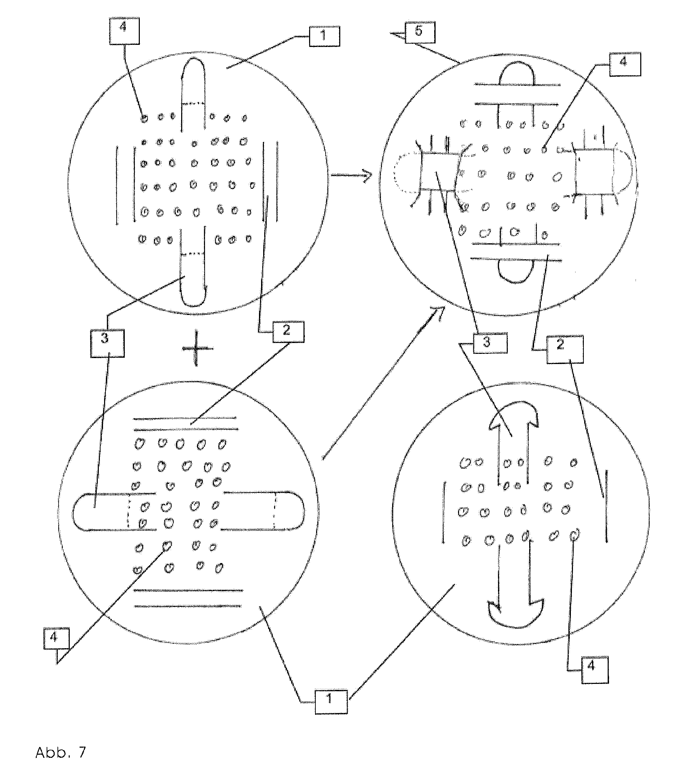

Geeignete lösliche Verbindungsmittel umfassen insbesondere wasserlösliche bzw. in physiologischen Medien lösliche Kleber, wie beispielsweise physiologisch verträgliche Sprühkleber oder Sprühpflaster, insbesondere solche auf Basis von Gelatinelösungen, Chitosanlösungen, oder Lösungen gelbildender Hydrokolloide auf Basis von Natrium- oder Kalium-Alginaten sowie Mischungen davon, wie insbesondere Mischungen aus Gelatine und Chitosan oder aus Gelatine und Alginaten; aber auch im Körper abbaubare, resorbierbare (verstoffwechselbare) Materialien, wie chirurgisches Nahtmaterial, resorbierbare Fäden oder Klammern (sogenannte Staples), resorbierbare Dübel, Nieten oder Schrauben etc.; sowie in den Biomatrixschichten angebrachte bzw. ausgebildete Laschen und Schlaufen, mittels denen zwei jeweils benachbarte Biomatrixschichten miteinander verbunden oder verknüpft werden können wie in der Abbildung 7 beispielhaft dargestellt. Es können auch unterschiedliche Verbindungsmittel miteinander kombiniert werden.

Derartige lösliche Verbindungsmittel sind insofern bevorzugt, als dadurch eine leichte Ablösung der oberen, der Wundseite abgewandten Schichten im Verlauf der Wundheilung erfolgen kann, wie vorstehend bereits im Detail ausgeführt.

Aus vorstehend dargestellten Gründen weisen die zur Bildung der erfindungsgemäßen Wundbehandlungsmaterialien verwendeten Biomatrixschichten Perforationen auf.

Eine Perforation im Sinne der vorliegenden Erfindung bezeichnet eine Durchlochung der schichtförmigen Biomatrixmaterialien. Dabei handelt es sich bei einer derartigen Durchlochung insbesondere um eine gezielte Einbringung von Löchern oder Schlitzen bzw. um durch die Dicke des Materials durchgehende Kanäle oder Durchgänge mit einer im Wesentlichen regelmäßigen Anordnung, Menge, Form und Größe.

Derartige Perforationen können als Schlitze oder Löcher ausgestaltet sein und weisen eine im wesentlichen dreieck-, viereck-, vieleck-, waben-, kreis- oder ellipsenförmige Ausgestaltungen auf.

Dabei umfasst im Sinne der vorliegenden Erfindung eine kreis- oder ellipsenförmige Ausgestaltung prinzipiell auch ovale geometrische Formen und eine viereckige Ausgestaltung der Perforationen umfasst prinzipiell alle bekannten geometrischen Viereck-Formen. Insbesondere sind darunter Vierecke mit sich gegenüberliegenden parallelen und gleich langen Seiten, wie Parallelogramme, insbesondere gleichwinkelige Parallelogramme wie Rechtecke oder Quadrate, sowie Rauten oder Rhomben oder Trapeze erfasst.

Darüber hinaus können jedoch auch Perforationen in jeglicher anderen denkbaren geometrischen Form oder auch in Phantasieformen gebildet werden. So ist beispielsweise ebenfalls denkbar, Perforationen in optisch bzw. ästhetisch ansprechenden Formen auszugestalten wie z.B. herz- oder sternförmig etc..

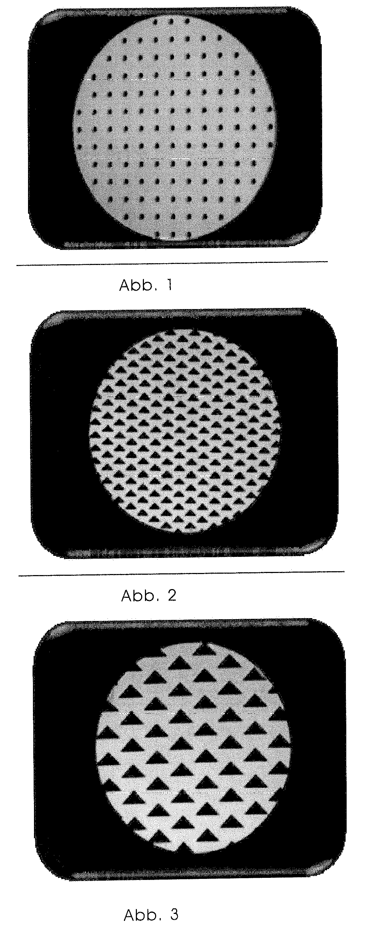

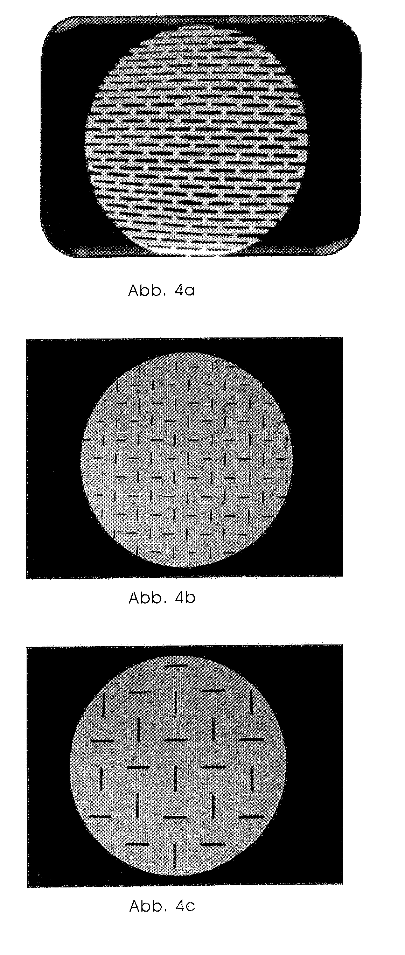

Bevorzugt sind runde und dreieckige Perforationen, wie beispielhaft in den Abbildungen 1 bis 3 dargestellt, sowie Perforationen in Form von Schlitzen oder in viereckiger Ausgestaltung. Letztere können insbesondere auch zueinander versetzt angeordnet sein, wodurch ein Muster entsteht, das mit einem Mauerverband nach Art eines Läufer- oder Binderverbandes, vergleichbar ist. Eine derartige Ausführungsform ist beispielhaft in Abbildung 4a dargestellt. In einer weiteren Ausführungsform können die Perforationen in Form von Schlitzen oder in viereckiger Ausgestaltung auf einer Biomatrixschicht auch zueinander um jeweils 90° versetzt angeordnet sein, wodurch ein Muster entsteht, wie beispielhaft in Abbildung 4b und 4c dargestellt.

Die Größe der Perforationen beträgt ca. 1 bis 15 mm, bevorzugt ca. 2 bis 10 mm, bevorzugter ca. 2 bis 8 mm. Bevorzugt sind runde Perforationen von 2 bis 8 mm Durchmesser, sowie dreieckige Perforationen mit 4 bis 10 mm Seitenlänge der Dreiecksperforation. Außerdem bevorzugt sind viereckige Perforationen mit Seitenlängen von 0,5 - 3 mm x 4 - 10 mm, bevorzugt 1 - 2 mm x 8 - 10 mm welche insbesondere im Muster eines Mauerverbands oder jeweils um 90° zueinander versetzt angeordnet sind.

Eine derartige Perforation der Biomatrixschichten gewährleistet einen guten Exsudatabfluss mit einem geringeren Druckverlust sowie eine bessere Verformbarkeit und damit Applizierbarkeit der Wundbehandlungsmaterialien.

Damit der Exsudatabfluss gewährleistet ist, ist es maßgeblich, dass die perforierten Biomatrixschichten derart miteinander verbunden sind, dass durch das Wundbehandlungsmaterial durchgehende Perforationen vorhanden sind. In solchen Ausführungsformen weisen somit auch möglicherweise vorhandene Klebeschichten eine entsprechende Perforation auf und es liegen durchgängige Perforationen durch die Gesamtschichtdicke des Wundbehandlungsmaterials vor.

Insbesondere bei Verwendung von löslichen Verbindungsmitteln ist es maßgeblich, dass die perforierten Biomatrixschichten derart miteinander verbunden sind, dass bei Auflösung des Verbindungsmittels durch das Wundbehandlungsmaterial durchgehende Perforationen gebildet werden. In solchen Ausführungsformen weisen möglicherweise vorhandene Klebeschichten zunächst im trockenen Zustand der Wundbehandlungsmaterialien keine entsprechende Perforation auf und im Kontakt mit dem Wundmilieu bzw. Exsudat wird die unperforierte Klebeschicht soweit aufgelöst, dass durchgängige Perforationen durch die Gesamtschichtdicke des Wundbehandlungsmaterials quasi in-situ gebildet werden.

Entsprechende erfindungsgemäße Ausführungsformen betreffen Wundbehandlungsmaterialien, worin die perforierten Biomatrixschichten derart miteinander verbunden sind, dass durch das Wundbehandlungsmaterial durchgehende Perforationen vorhanden sind, sowie Wundbehandlungsmaterialien, worin die perforierten Biomatrixschichten derart miteinander verbunden sind, dass bei Auflösung des Verbindungsmittels durchgehende Perforationen gebildet werden.

Weitere Ausführungsformen betreffen Wundbehandlungsmaterialien, worin die Biomatrixschichten derart aufeinander angeordnet sind, dass auch bei Auflösen des Verbindungsmittels und Verschieben der Biomatrixschichten aufeinander durch das Wundbehandlungsmaterial durchgehende Perforationen erhalten bleiben.

Ein Erhalt der durchgehenden Perforationen durch die Gesamtdicke des Wundbehandlungsmaterials auch bei Auflösung des Verbindungsmittels und Verschieben der einzelnen Biomatrixschichten aufeinander ist maßgeblich um auch dann einen optimalen Exsudatabfluss zu gewährleisten, da beim Verschieben der perforierten Schichten die Löcher so gegeneinander verschoben werden können, dass die durch das Wundbehandlungsmaterial durchgehenden Kanäle verschlossen werden. Dies kann durch geeignete Wahl der Geometrien der Perforationslöcher bzw. der Perforationsmuster sowie geeignete Anordnung der perforierten Biomatrixschichten zueinander sowie insbesondere auch durch Kombination von Biomatrixschichten mit jeweils unterschiedlich großen Perforationen erreicht werden.

Beispielsweise können Biomatrices mit unterschiedlich großen dreieckigen oder rechteckigen Löchern miteinander verbunden werden, oder Biomatrixschichten mit rechteckigen, schlitzförmigen Perforationen werden um 90° versetzt übereinander angeordnet.

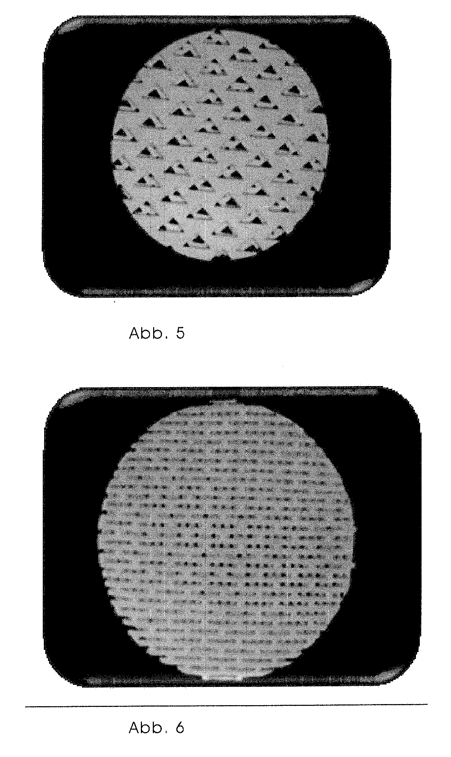

Beispielsweise kann eine Schicht vier Löcher aufweisen, eine darüber liegende Schicht beispielsweise fünf Löcher, so dass beim Verschieben das fünfte Loch wiederum eine Durchtrittsmöglichkeit zu einem der anderen Löcher in der unterliegenden Schicht hat. Eine weitere Möglichkeit besteht darin, dass die Perforationen in den verschiedenen Schichten unterschiedliche Größen aufweisen, beispielsweise kann eine Schicht kleinere, z.B. Dreiecke, aufweisen und die darüber liegende Schicht größere Dreiecke, so dass auch hier beim Verschieben Durchtrittsmöglichkeiten erhalten bleiben. Beispielhafte Ausführungsformen hierzu zeigen die Abbildungen 5 und 6.

Die erfindungsgemäßen Wundbehandlungsmaterialien sind prinzipiell erhältlich durch Bereitstellen der perforierten Biomatrixschichten und Verbinden der gewünschten Anzahl davon in der geeigneten Anordnung mittels einem oder mehreren geeigneten erfindungsgemäßen Verbindungsmitteln unter Bildung der geschichteten Wundbehandlungsmaterialien oder durch Verbinden der gewünschten Anzahl geeigneter Biomatrixschichten und anschließendem Anbringen der durchgehenden Perforationen unter Bildung der Wundbehandlungsmaterialien mit durchgehenden Perforationen.

In den erfindungsgemäßen Verfahren werden die Perforationen als Schlitze oder Löcher ausgestaltet und weisen im Wesentlichen kreis-, dreieck-, viereck-, waben- oder ellipsenförmige Formen auf und können prinzipiell mit üblichen Perforationsverfahren wie Schneiden oder Stanzen ausgebildet werden. Schneiden kann beispielsweise durch geeignete Messer oder Schneidwerkzeuge wie z.B. Rollenstanzen oder auch mittels Laserschneiden erfolgen. Bevorzugt werden die Perforationen mittels Stanzen oder mittels Laser auf bzw. in den Biomatrixschichten angebracht werden. Auf gleiche Weise können auch die als Verbindungsmittel genutzten Laschen und Schlaufen in den Biomatrixschichten gebildet werden.

Zur Herstellung der erfindungsgemäßen Wundbehandlungsmaterialien wird entweder erst die gewünschte Anzahl unperforierter Biomatrixschichten mittels des gewünschten Verbindungsmittels, insbesondere einem Kleber, miteinander verbunden und anschließend die Perforationen auf bzw. in dem so erhaltenen geschichteten Materal angebracht, daß diese durch die Gesamtdicke durchgängige Öffnungen oder Kanäle bilden.

Alternativ werden zunächst die Perforationen auf bzw. in den einzelnen Biomatrixschichten angebracht und diese anschließend in der gewünschten Anzahl und mittels des gewünschten Verbindungsmittels miteinander verbunden.

Dies ist insbesondere dann vorteilhaft, wenn als Verbindungsmittel in den Biomatrices ausgebildete Laschen und Schlaufen gewählt werden, durch die die einzelnen Biomatrixschichten quasi miteinander verknüpft werden.

Die Erfindung umfasst damit insbesondere ein Verfahren zur Herstellung eines bevorzugten erfindungsgemäßen Wundbehandlungsmaterials, umfassend die Schritte
a1) Aufbringen eines Klebers auf eine Biomatrixschicht und Aufbringen einer weiteren Biomatrixschicht auf der mit Kleber beschichteten ersten Biomatrixschicht, gegebenenfalls Aufbringen weiterer Kleber- und Biomatrixschichten bis zum Erhalt der gewünschten Gesamtdicke des Wundbehandlungsmaterials und Perforation der miteinander verbundenen Biomatrixschichten;
   oder
a2) Perforation der Biomatrixschichten, Aufbringen eines Klebers auf eine perforierte Biomatrixschicht und Aufbringen einer weiteren perforierten Biomatrixschicht auf der mit Kleber beschichteten ersten Biomatrixschicht, gegebenenfalls Aufbringen weiterer Kleber- und Biomatrixschichten bis zum Erhalt der gewünschten Gesamtdicke des Wundbehandlungsmaterials, wobei die perforierten Biomatrixschichten so angeordnet werden, dass bei Auflösung des Klebers durch die Gesamtdicke des Wundbehandlungsmaterials durchgehende Perforationen gebildet werden,
b) gegebenenfalls Trocknen des Klebers; sowie
c) Sterilisieren und/oder Konfektionieren.
Bevorzugt ist darunter die Variante a2).

Das Verfestigen des Klebers erfolgt in der Regel durch Trocknen oder UVinduzierte Vernetzungsreaktionen oder andere bekannte Verfahren. Bevorzugt erfolgt ein Trocknen des Klebers, insbesondere bei Verwendung von Gelatinelösungen oder Chitosanlösungen, in der Regel bei Raumtemperatur (20 °C), wobei auch bei höheren Temperaturen bis ca. 80 °C getrocknet werden kann. Maßgeblich ist hierbei, daß durch die Trocknungstemperatur keine thermalen Schäden an den Biomatrices auftreten, entsprechend müssen temperaturempfindliche Materialien bei geringeren Temperaturen getrocknet werden, wohingegen unempfindlichere Materialien auch bei höheren Temperaturen getrocknet werden können.

Die Sterilisation und die Konfektionierung der erfindungsgemäßen Wundbehandlungsmaterialien kann nach bekannten und gebräuchlichen Methoden durchgeführt werden.

Gegenstand der Erfindung sind außerdem die nach dem vorstehenden Verfahren erhältlichen Wundbehandlungsmaterialien.

Die erfindungsgemäßen Wundbehandlungsmaterialien können außerdem eine oder mehrere Deckschichten synthetischer oder semi-synthetischer Wundbehandlungsmaterialien aufweisen. Derartige Ausführungsformen betreffen Kombinationsmaterialien, worin neben den erfindungsgemäßem Biomatrixmaterial auch herkömmliche Wundverbandsmaterialien und synthetische Materialien, wie PVA-, PU- oder PE-Schäume oder Gazen verwendet werden, insbesondere wird hierbei im unteren Bereich der Wunde (wundseitig) das erfindungsgemäße Biomatrixschicht-Material eingesetzt und im oberen Bereich mit den herkömmlichen synthetischen oder semi-synthetischen Materialien aufgefüllt. Dies ist insbesondere dann vorteilhaft, wenn besonders tiefe Wunden behandelt werden, um kostengünstige Wundbehandlungslösungen zu erzielen.

Insbesondere betrifft die vorliegende Erfindung auch die erfindungsgemäßen Wundbehandlungsmaterialien zur Verwendung als Mittel zur Blutstillung sowie in der Behandlung von akuten Wunden wie z.B. traumatischen oder chirurgischen Wunden, z.B. Tumorwunden, sowie zur Behandlung von chronischen Wunden wie z.B. Dekubitus, Ulcus cruris, diabetischem Fußsyndrom etc., insbesondere zur Behandlung von exsudierenden und stark exsudierenden Wunden, zur Verwendung als Implantat (zum Verbleib im Körper) sowie zur Verwendung in der Vakuum-unterstützten Wundbehandlungstherapie.

Besonders bevorzugt ist die Verwendung in der Behandlung chronischer und exsudierender Wunden sowie in der Vakuum-unterstützten Wundbehandlungstherapie.

Die erfindungsgemäßen schichtförmigen Biomatrices können dabei pharmazeutische Produkte oder Medizinprodukte darstellen.

Die Anwendung erfolgt dabei in der Regel so, dass die erfindungsgemäßen Wundbehandlungsmaterialien auf die zu behandelnden Körperpartien oder in der Wunde trocken aufgebracht und entweder durch das vorhandene Wundsekret oder mit Wasser bzw. einer wässrigen Lösung oder physiologischer Kochsalzlösung, die einen oder mehrere Wirkstoffe und/oder einen oder mehrere Hilfsstoffe enthalten kann, durchfeuchtet und rehydratisiert wird. Es ist jedoch auch möglich, die erfindungsgemäßen Wundbehandlungsmaterialien vor dem Aufbringen auf die zu behandelnde Körperpartie anzufeuchten.

Die erfindungsgemäßen Wundbehandlungsmaterialien sind insbesondere für die Verwendung in der Vakuum-unterstützten Wundbehandlung geeignet und vorgesehen. Die Vakuumtherapie, auch bezeichnet als Unterdrucktherapie, Vakuumversiegelung, Topische Negative Drucktherapie (TNP), "Negative Pressure Wound Therapy" (NPWT), oder "Vacuum Assisted Closure-Therapy (VAC), stellt ein bekanntes und weltweit angewandtes Verfahren zur Wundheilung dar, worin ein okklusiver Wundverschluss mit einem Abtransportsystem von Wundflüssigkeit (Exsudat) kombiniert wird. Durch die Ausübung eines Unterdrucks oder Vakuums auf die Wunde wird die Wunde verkleinert, die Wundränder ziehen sich zusammen, die Wundheilung wird beschleunigt und störendes Wundsekret wird aus der Wunde wieder entfernt. Die erfindungsgemäße Verwendung in der Vakuum-Therapie erfolgt dabei grundsätzlich nach den allgemein bekannten und etablierten Verfahren.

Insbesondere erfolgt im Rahmen der Vakuum-unterstützten Wundbehandlung die Anwendung der erfindungsgemäßen Wundbehandlungsmaterialien in der Regel so, dass das erfindungsgemäße Wundbehandlungsmaterial in die Wunde eingebracht wird, wobei das Material gegebenenfalls auf die Form der Wunde zugeschnitten wird. Gegebenenfalls wird mit herkömmlichem Wundverbandmaterial oder synthetischem bzw. semi-synthetischem Füll- oder Deckmaterial aufgefüllt. Anschließend wird die Wunde luftdicht mit einer vakuumfesten Abdeckungsfolie verschlossen. Derartige vakuumfeste Abdeckungsfolien sind ebenfalls bekannt und etabliert und können außerdem geeignete Vorrichtungen zum Anbringen von Drainageschläuchen (beispielsweise auch solche, die kommerziell als T.R.A.C. Pad bezeichnet werden), geeignete Abdichtpasten und/oder Abdichtfolien sowie gegebenenfalls direkt auch die Drainageschläuche zum Abführen der Wundflüssigkeit (Drainageeinheit) sowie zum Anschließen der Auffangbehälter oder Auffangsysteme für die abgesaugte Wundflüssigkeit sowie die Unterdruck-erzeugenden Pumpen, u.ä. aufweisen.

Erfindungsgemäß umfasst sind außerdem Wundbehandlungsmittel umfassend ein erfindungsgemäßes Wundbehandlungsmaterial sowie eine vakuumfeste Abdeckungsfolie, gegebenenfalls in Kombination mit der Vorrichtung zum Anbringen der Drainageeinheit. Dabei können das erfindungsgemäße Wundbehandlungsmaterial und die vakuumfeste Abdeckungsfolie, gegebenenfalls mit der Drainageeinheit, als fester Verbund oder einzeln in Form einer sogenannten Kombinations- oder Kit-of-parts-Anordnung ausgestaltet sein.

Neben der Anwendung im Bereich der Vakuum-Therapie sind die erfindungsgemäßen Wundbehandlungsmaterialien auch für Wunden vorteilhaft, die in herkömmlicher Weise, d.h. nur durch den Einsatz der Auflage als solche, verheilen. Sowohl bei dieser herkömmlichen Behandlung als auch in der Vakuum-Therapie ist das medizinische Pflegepersonal durch einfaches Entfernen einzelner, nicht eingeheilter Lagen in der Lage, einen schnellen Überblick über den Fortschritt der Wundheilung zu erhalten, ohne die Wunde zu traumatisieren.

Die Erfindung wird durch die folgenden Beispiele näher veranschaulicht. Die Beispiele stellen lediglich Exemplifizierungen dar und der Fachmann ist in der Lage, die spezifischen Beispiele auf weitere beanspruchte Ausführungsformen auszudehnen.

### BEISPIELE

### Beispiel1 Ausführunasbeispiete

| **Nr.** | **Biomatrix** | **Anzahl der Schichten** | **Dicke der Schichten** | **Gesamtdicke** | **Verbindungsmittel** | **Perforation** |
|---|---|---|---|---|---|---|
| 1a | Collagen * | 1 | 2 mm | 2 mm | - | rund, Ø 3 mm (Abb. 1; 13) |
| 1b | Collagen * | 1 | 4 mm | 4mm | - | rund, 02mm |
| 1c | Collagen * | 1 | 6 mm | 6 mm | - | rund, Ø 2 mm |
| 1d | Collagen * Epoxidvernetzt (5%) | 2 | je 1 mm | 2 mm | - | rund, Ø 2 mm (Abb. 12) |
| 2 | Collagen * | 1 | 2 mm | 2 mm | - | dreieckig, Länge 4 mm (Abb. 2) |
| 3 | Collagen * | 1 | 2 mm | 2 mm | - | dreieckig, Länge 8 mm (Abb. 3) |
| 4a | Collagen * | 1 | 2 mm | 2 mm | - | viereckig, 1 x 10 mm (Abb. 4a) |
| 4b | Collagen * | 1 | 2mm | 2 mm | - | viereckig, 0,5 x 5 mm (Abb. 4b) |
| 4c | Collagen * | 1 | 2 mm | 2 mm | - | viereckig, 1 x 10 mm (Abb. 4c) |
| 5 | Collagen * | 2 | je 2 mm | 4 mm | Gelatinekleber | dreieckig, 4 und 8 mm (Abb. 5) |
| 6a | Collagen * | 4 | je 2 mm | 8mm | Gelatinekleber | viereckig, 1 x 10 mm 90° Versatz (Abb. 6; 14) |
| 6b | Collagen * | 4 | je 2 mm | 8 mm | Gelatinekleber | rund, Ø 2 mm (Abb. 15) |
| 7 | Collagen-Alginat-Mischung* | 1 | 2 mm | 2 mm | - | rund, Ø 2 mm |
| 8 | Collagen-Alginat-Mischung* | 2 | je 2 mm | 4 mm | Gelatinekleber | rund, Ø 2 mm |
| 9 | Collagen * | 2 | je 4 mm | 8mm | Chitosan-kleber | rund, Ø 2 mm |
| 10 | Collagen * | 2 | je 4 mm | 8 mm | Gelatine-/Chitosan-Kleber** | rund, Ø 2mm |
| 11 | Collagen * | 2 | je 4 mm | 8 mm | Gelatine-/Alginat-Kleber** | rund, Ø 2mm |
| 12 | Collagen * | 2 | je 4 mm | 8 mm | Sprühkleber*** | rund, Ø 2 mm |

| | | | | | | |
|---|---|---|---|---|---|---|
| * Poröse Biomatrix, erhältlich durch Gefriertrocknung einer Biomatrix-Suspension; ggf. mittels Epoxid chemisch quervernetzt; ** wässriger Gelatine-/Chitosan- bzw. Gelatine-/Natriumalginat-Kleber (Gew.-Verhältnis 2:1) ***Hansaplast® Sprühpflaster, Beiersdorf (Copolymerisat auf Methacrylatbasis, Ethylacetat, n-Pentan) | | | | | | |

Die lochartigen Perforationen wurden mittels Stanzen sowie mittels Laser angebracht.

Die Biomatrixschichten bzw. Wundbehandlungsmaterialien können außerdem weitere Wirk- und Hilfsstoffe umfassen oder durch Variation der Schichtdicke und/oder der Anzahl der Biomatrixschichten insgesamt dicker oder dünner hergestellt werden.

### Beispiel 2 Untersuchung im Wundsimulator

### 1. Versuchsaufbau

Zentraler Baustein ist ein Aluminiumblock (Maße: 150 x 140 x 60 mm), mit einer 2 cm tiefen, kreisrunden Einfräsung mit einem Durchmesser von 10 cm. Um den Simulatorblock im Wasserbad zu temperieren wurde jeweils an zwei gegenüberliegenden Seiten eine Winkelplatte aus Edelstahl befestigt, diese können so mit den Wänden des Wasserbades verschraubt werden, dass der Simulatorblock im Wasser eingelassen ist. Auf der Unterseite des Blocks befindet sich eine Bohrung für die Modellflüssigkeitszufuhr, an der Vorderseite des Blocks ist die Bohrung für die Druckmessung platziert. Der Aluminiumblock wird durch das Wasserbad auf 35 - 37°C erwärmt, um die Hauttemperatur zu simulieren.

Die Einfräsung ist nachfolgend als Simulatorkammer bezeichnet. Diese wird mittels dünner Polymerfolie, die selbstklebend ist, luftdicht verschlossen. Für die Druckanlegung wird eine herkömmliche Vorrichtung in Form eines kommerziell erhältlichen sogenannten T.R.A.C Pads mit der Folie verklebt.

Die Vakuumpumpe zur Regulierung des Simulatorkammerdrucks ist eine PTFE Membranpumpe (Typ V-700; Büchi).
Die Druckmessung erfolgt mit einem handelsüblichen Differenzdruckmanometer.
Die Modelflüssigkeit wird über eine handelsübliche Schlauchpumpe vom Vorratsgefäß in die Simulatorkammer gefördert, die Aufzeichnung des Simulatorkammerdrucks erfolgt am PC über die Manometersoftware.

Das Auffanggefäß für die Modelflüssigkeit wird permanent über eine Präzisionswaage (CP 4202 S, Sartorius) ausgewogen um den Absaugfluss zu definieren, die Aufzeichnung der Daten erfolgt am PC mittels Sarto Collect Software von Sartorius.

### 2. Versuchsdurchführung

### 2.1 Testphase des Wundsimulators

Für die allgemeine Funktionsüberprüfung wurde der Teststand ohne Probe, nur bestückt mit einer Aluminiumunterlegscheibe als Füllmaterial und einer Duran Glasfritte für die gleichmäßige Verteilung der Modellflüssigkeit in der Kammer, gestartet. Somit konnte eine Aussage darüber getroffen werden, in wie weit die Stabilität des angelegten Vakuums in der Kammer von den Füllmaterialien beeinflusst wird.

### 2.2 Vergleichsversuch mit herkömmlichen Wundauflagen

Im zweiten Schritt der Testphase wurde eine herkömmliche, in der Vakuum-Therapie etablierte Wundauflage auf Basis eines PU-Schaums (V.A.C. GranuFoam) der Firma KCl® sowie eine herkömmliche Gaze der Firma Kalff® untersucht. Weitere Messungen wurden mit herkömmlichen Wundbehandlungsmaterialien auf Basis gefriergetrockneter poröser Collagenmaterialien (Matristypt®) der Firma Dr. Suwelack Skin & Health Care AG, wobei es sich um einstückige (ungeschichtete), unperforierte schwammartige Materialien handelt, durchgeführt.

Dafür wurde eine Albuminlösung (4,04 g Bovine Serum Albumine von Prolabo in 100mL 0,9%iger Natriumchloridlösung) mit einer Viskosität von 20 mPas als Modelflüssigkeit verwendet. Im nächsten Schritt wurde dann die Albuminmodellflüssigkeit auf eine Viskosität von ca. 100 mPas angepasst, dafür wurden verschiedenste Lösungen zur Viskositätsanpassung, bestehend aus Xanthan, Hyaluronsäure bzw. Carbopol Ultrez 21 (Polyacrylsäure) angesetzt und getestet.

### 2.3 Versuche mit erfindungsgemäßen Wundbehandlungsmaterialien

Es wurden verschiedene perforierte Biomatrixschichten hinsichtlich ihrer Eignung zur Bildung der erfindungsgemäßen Wundbehandlungsmaterialien sowie aus solchen Biomatrixschichten zusammengesetzte Wundbehandlungsmaterialien ausgewählt aus Beispiel 1 im Wundsimulator untersucht.

### 3. Ergebnisse

### 3.1 Testlauf ohne Wundauflage

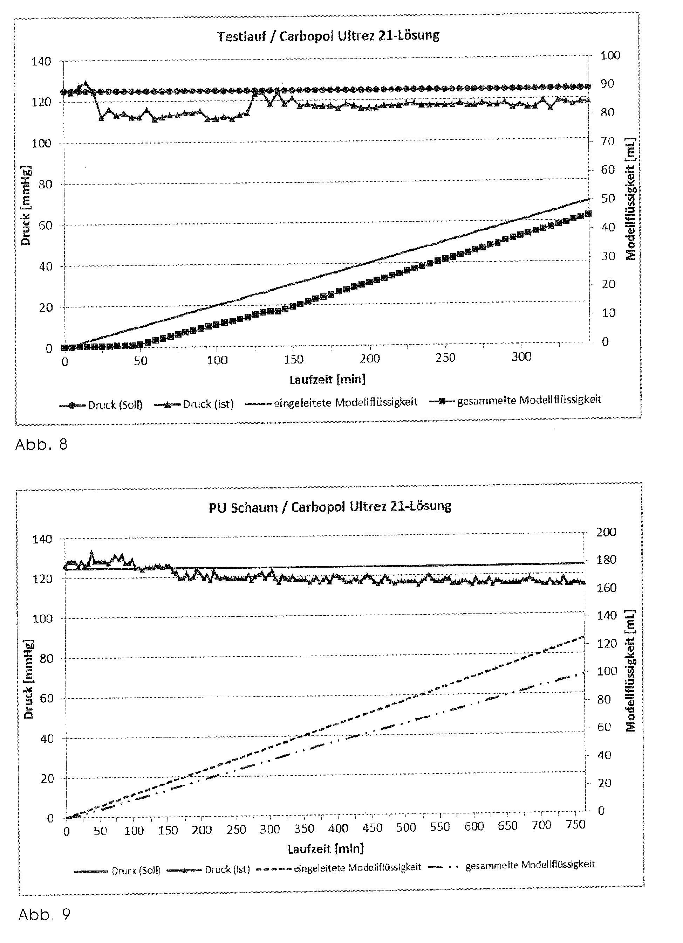

Abbildung 8 zeigt exemplarisch die Datenaufzeichnung vom Kammerinnendruck und die Mengen an eingeleiteter und abgesaugter Modellflüssigkeit über einen Zeitraum von 345 min. Erkennbar ist, dass der gemessene Innendruck mit einem Durchschnittswert von 117 mmHg nur wenig vom eingestellten Druck abweicht. Für die vorherigen Testläufe mit den Modellflüssigkeiten, Albumin, Xantan und Hyaluronsäure sehen die Kurvenverläufe ähnlich aus. Als wichtige Aussage daraus ist zu entnehmen, dass für die weiteren Testläufe mit der jeweiligen Wundauflage keine Beeinflussung auf Kammerinnendruck bzw. Flüssigkeitsabtransport von den Füllmaterialien (Aluminiumunterlegscheibe, Glasfritte) ausgehen.

### 3.2 Herkömmliche Materialien

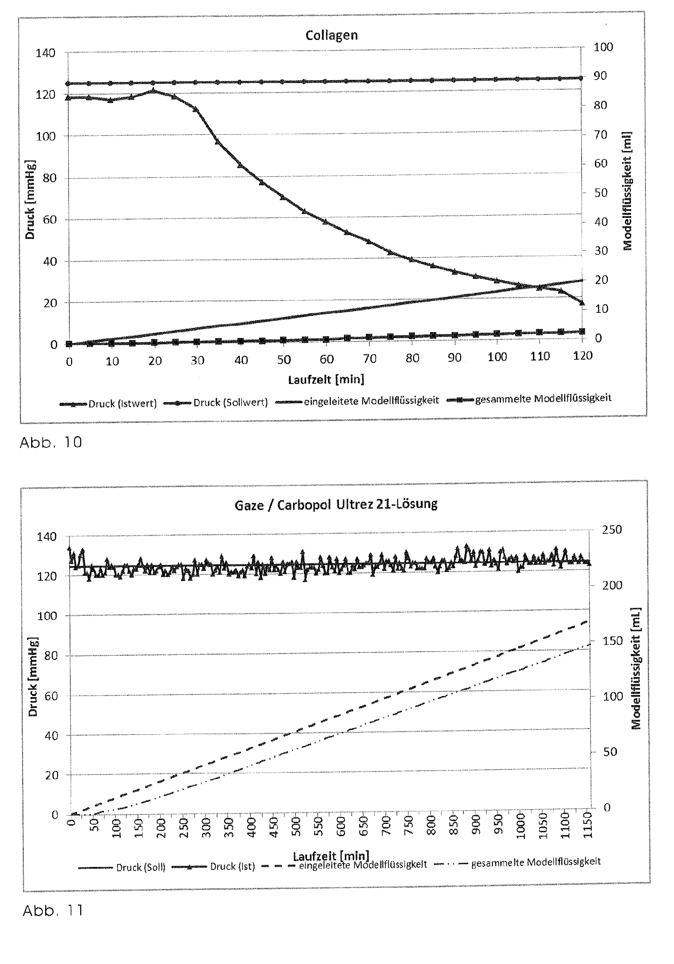

Die Abbildungen 9, 10 und 11 zeigen die Ergebnisse der Untersuchung herkömmlicher Wundauflagen aus PU-Schaum, Gaze und Collagenschwamm im Wundsimulator.
Beim Collagenschwamm wurde ein starker Druckabfall gemessen. Das Collagen blockiert den Modellflüssigkeitsabtransport und verhindert einen konstanten Druckaufbau in der Simulatorinnenkammer. Der PU-Schaum zeigt über die gesamte Messzeit von 795 min einen konstanten Druck mit 120 mmHg (Durchschnitt) bei einem angelegten Unterdruck von 125mmHg und auch der Flüssigkeitsabtransport ist mit leichter Abschwächung konstant, der durchschnittliche Fluss liegt bei 0,13 ml/min. Die Gaze zeigt wie auch der PU-Schaum über die Messzeit von 1160 min einen konstanten Unterdruck in der Kammer. Mit durchschnittlich 124 mmHg liegt der Druck beim angelegten Unterdruck von 125 mmHg. Die Schwankungen sind durch den Pumpentyp bedingt. Der Flüssigkeitsabtransport ist gleichmäßig über die gesamte Laufzeit.

### 3.3 Erfindungsgemäße Wundbehandlungsmaterialien

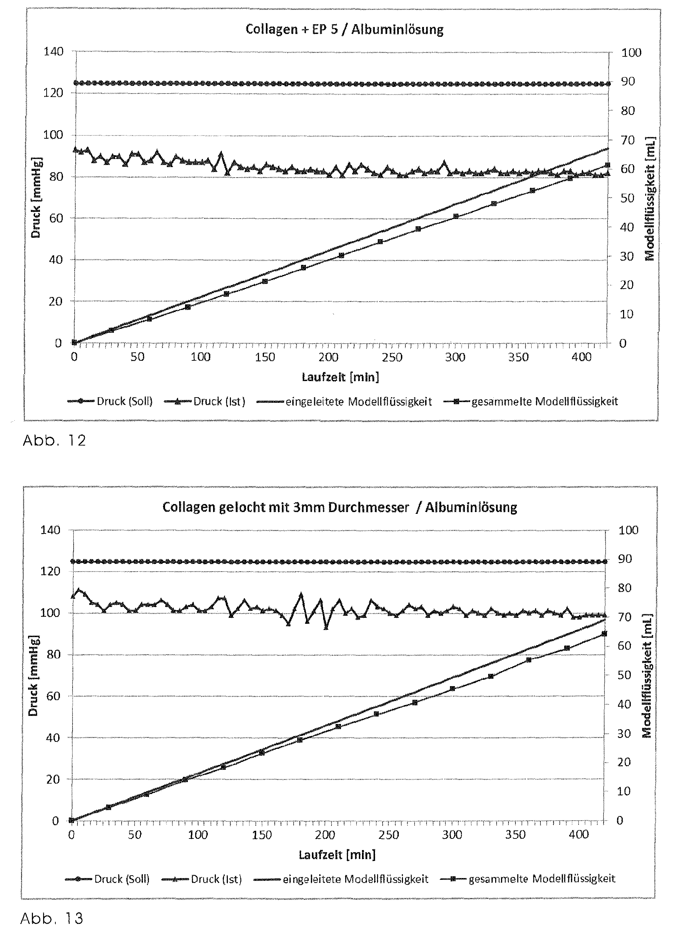

Abbildung 12 zeigt exemplarisch die Datenaufzeichnung vom Kammerinnendruck und die Mengen an eingeleiteter und abgesaugter Modellflüssigkeit über einen Zeitraum von 420 min bei Verwendung einer perforierten Collagen-Biomatrixschicht nach Beispiel 1 Nr.1d (gefriergetrocknetes Collagen mittels Epoxid (5%) quervernetzt).

Erkennbar ist, dass sich der Unterdruck in der Simulatorkammer im Vergleich zum einstückigen, unperforierten herkömmlichen Collagenmaterial wesentlich verbessert hat. Mit durchschnittlich 85 mmHg bei angelegten 125 mmHg ist jedoch eine Abweichung von 40 mmHg ermittelt worden. Im Vergleich mit dem PU-Schaum, ist dies als schlecht zu bewerten und bedarf einer Optimierung. Der Flüssigkeitsabtransport ist vergleichbar mit dem Verhalten des PU-Schaums und hat sich demnach stark verbessert zum herkömmlichen Collagenmaterial.

Abbildung 13 zeigt die Ergebnisse einer Biomatrixschicht nach Beispiel 1 Nr. 1 a.

Dabei zeigt sich, dass sich der Unterdruck in der Simulatorkammer dem angelegten Unterdruck der Pumpe angeglichen hat. Bei angelegtem Unterdruck von 125 mmHg wurde im Durchschnitt 102 mmHg über den Messzeitraum von 420 min gemessen. Die Druckdifferenzen konnten also von 40 mmHg auf 27 mmHg reduziert werden. Dies ist darauf zurückzuführen, dass durch das Lochen der Collagensheets Kanäle existieren, die einen gleichmäßigen Unterdruckaufbau und gleichzeitig auch Flüssigkeitsabtransport ermöglichen.

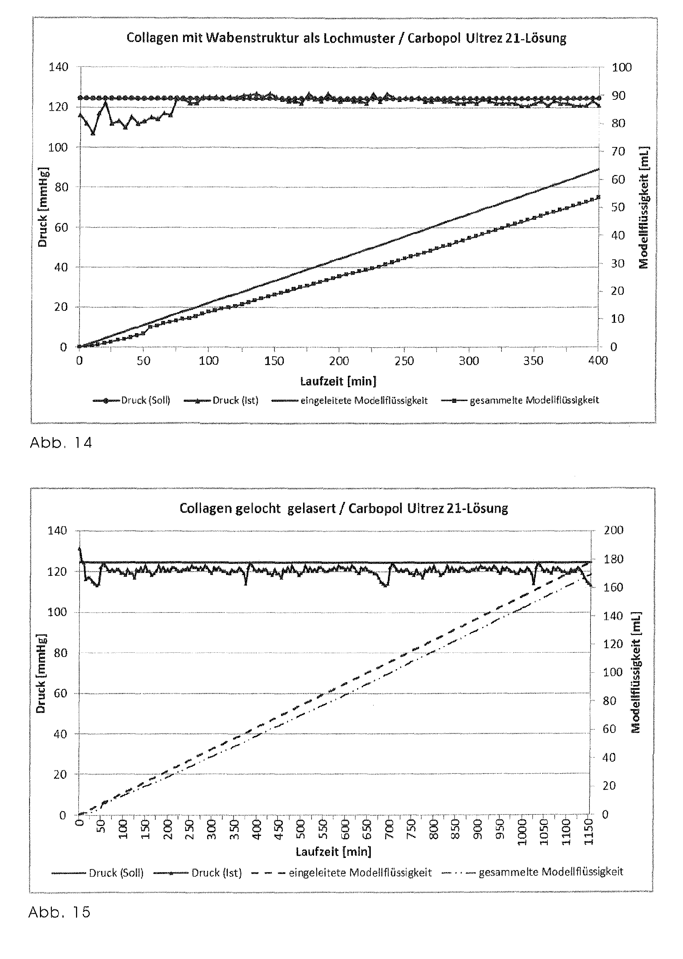

Abbildungen 14 und 15 zeigen die Ergebnisse von Materialien nach Beispiel 1 Nr. 6a und 6b.

Dabei zeigt sich, dass die Geometrie der Löcher dabei nicht wesentlich eine Rolle spielt. Wichtig ist, dass durchgängige Kanäle in der Wundauflage vorliegen und diese auch durch mögliche Verschiebung der Wundauflage nicht verstopft werden.

Auch die Variation der Gesamtschichtdicke der Wundbehandlungsmaterialien bis ca. 8 mm wirkt sich nicht wesentlich auf den Unterdruck in der Simulatorkammer und den Abtransport der Modellflüssigkeit aus.

### ERLÄUTERUNG ZU DEN ABBILDUNGEN

Die Abbildungen 1 bis 15 erläutern beispielhaft den Gegenstand der vorliegenden Erfindung und stellen verschiedene mögliche Ausgestaltungen und Ausführungsformen der erfindungsgemäßen Wundbehandlungsmaterialien bzw. der perforierten Biomatrixschichten, die diese bilden, dar.

Abbildung 1 zeigt eine beispielhafte Ausführungsform einer perforierten Biomatrixschicht mit runden bzw. kreisförmigen Perforationen.

Abbildung 2 zeigt eine beispielhafte Ausführungsform einer perforierten Biomatrixschicht mit dreieckigen Perforationen (Seitenlänge 4 mm).

Abbildung 3 zeigt eine beispielhafte Ausführungsform einer perforierten Biomatrixschicht mit dreieckigen Perforationen (Seitenlänge 8 mm).

Abbildung 4a zeigt eine beispielhafte Ausführungsform einer perforierten Biomatrixschicht mit viereckigen bzw. schlitzförmigen Perforationen im Muster eines Mauerversatzes.

Abbildung 4b zeigt eine beispielhafte Ausführungsform einer perforierten Biomatrixschicht mit schlitzförmigen Perforationen mit jeweils 90° Versatz.

Abbildung 4c zeigt eine beispielhafte Ausführungsform einer perforierten Biomatrixschicht mit schlitzförmigen Perforationen mit jeweils 90° Versatz.

Abbildung 5 zeigt eine beispielhafte Ausführungsform eines erfindungsgemäßen Wundbehandlungsmaterials aus zwei perforierten Biomatrixschichten mit unterschiedlichem Perforationsmuster.

Abbildung 6 zeigt eine beispielhafte Ausführungsform eines erfindungsgemäßen Wundbehandlungsmaterials aus vier perforierten Biomatrixschichten mit unterschiedlichem Perforationsmuster.

Abbildung 7 zeigt eine beispielhafte Ausführungsform, worin das Verbindungsmittel in Form von in den Biomatrixschichten ausgebildeten Laschen und Schlaufen ausgebildet ist.

Abbildung 8 zeigt das Ergebnis des Testlaufs ohne Wundauflage im Wundsimulator.

Abbildung 9 zeigt das Ergebnis einer herkömmlichen PU-Schaum Wundauflage im Wundsimulator.

Abbildung 10 zeigt das Ergebnis einer herkömmlichen Collagenschwamm Wundauflage im Wundsimulator.

Abbildung 11 zeigt das Ergebnis einer herkömmlichen Gaze Wundauflage im Wundsimulator.

Abbildung 12 zeigt das Ergebnis einer Wundauflage im Wundsimulator, worin zwei beispielhafte Ausführungsformen perforierter Biomatrixschichten mit runden bzw. kreisförmigen Perforationen ohne Verbindungsmittel geschichtet und untersucht wurden.

Abbildung 13 zeigt das Ergebnis einer beispielhaften Ausführungsform einer perforierten Biomatrixschicht mit runden bzw. kreisförmigen Perforationen im Wundsimulator.

Abbildung 14 zeigt das Ergebnis einer erfindungsgemäßen Wundauflage im Wundsimulator.

Abbildung 15 zeigt das Ergebnis einer erfindungsgemäßen Wundauflage im Wundsimulator.

In Abbildung 7 weisen die Bezugszeichen folgende Bedeutung auf:
- 1: Biomatrixschicht
- 2: aus dem Biomatrixmaterial gebildete Schlaufe
- 3: aus dem Biomatrixmaterial gebildete Lasche
- 4: Perforationen
- 5: Wundbehandlungsmaterial aus zwei über Schlaufen und Laschen verbundenen Biomatrixschichten

## Patentansprüche

1. Wundbehandlungsmaterial mit mindestens zwei perforierten Biomatrixschichten die mittels eines Verbindungsmittels miteinander verbunden sind.

2. Wundbehandlungsmaterial nach Anspruch 1, worin die perforierten Biomatrixschichten derart miteinander verbunden sind, dass durch das Wundbehandlungsmaterial durchgehende Perforationen vorhanden sind.

3. Wundbehandlungsmaterial nach Anspruch 1, worin die perforierten Biomatrixschichten derart miteinander verbunden sind, dass bei Auflösung des Verbindungsmittels durchgehende Perforationen gebildet werden.

4. Wundbehandlungsmaterial nach einem der vorhergehenden Ansprüche, worin die Biomatrixschichten aus strukturbildenden natürlichen Polymeren aus der Gruppe der tierischen und pflanzlichen Hydrokolloide und Mischungen davon gebildet sind.

5. Wundbehandlungsmaterial nach einem der vorhergehenden Ansprüche, worin die natürlichen Polymere ausgewählt sind aus der Gruppe der Collagene und der Polysaccharide wie aus der Gruppe der Alginate.

6. Wundbehandlungsmaterial nach einem der vorhergehenden Ansprüche, worin die Biomatrixschichten außerdem mindestens einen Wirk- und/oder Hilfsstoff enthalten, insbesondere mindestens ein Wirkstoff aus der Gruppe der blutstillenden Mittel und/oder aus der Gruppe der Wundbehandlungsmittel.

7. Wundbehandlungsmaterial nach einem der vorhergehenden Ansprüche, worin die Biomatrixschichten erhältlich sind durch ein Verfahren welches die Schritte umfasst:
a) Herstellen einer wässrigen Suspension oder einer Lösung mindestens eines strukturbildenden natürlichen Polymers;
b) gegebenenfalls Einmischen eines oder mehrerer Wirk- und/oder Hilfsstoffe;
c) Gießen der Mischung in eine geeignete Form;
d) Trocknen, vorzugsweise Gefriertrocknen, der Mischung unter Erhalt eines porösen Formkörpers;
e) gegebenenfalls Schneiden des aus Schritt d) erhältlichen Formkörpers in Schichten;
f) gegebenenfalls Schneiden der Schichten aus Schritt e) in die gewünschte geometrische Ausgestaltung der Biomatrixschicht und
g) gegebenenfalls Perforieren der Biomatrixschicht.

8. Wundbehandlungsmaterial nach einem der vorhergehenden Ansprüche, worin die Zusammensetzung und/oder die Perforation der Biomatrixschichten jeweils gleich oder verschieden ist.

9. Wundbehandlungsmaterial nach einem der vorhergehenden Ansprüche, worin die Schichtdicke der einzelnen Biomatrixschichten jeweils gleich oder verschieden ist und eine Schichtdicke von bis zu 10 mm aufweist und/oder das eine Gesamtdicke von mindestens 2 mm aufweist.

10. Wundbehandlungsmaterial nach einem der vorhergehenden Ansprüche, worin das Verbindungsmittel in Wasser oder physiologischem Milieu löslich ist und insbesondere ausgewählt ist aus der Gruppe umfassend wasserlösliche Kleber wie physiologisch verträgliche Sprühkleber, Gelatine und Chitosan, resorbierbare Fäden, resorbierbare Klammern oder in den Biomatrixschichten ausgebildete Laschen und Schlaufen.

11. Wundbehandlungsmaterial nach einem der vorhergehenden Ansprüche, worin die Biomatrixschichten derart aufeinander angeordnet sind, dass auch bei Auflösen des Verbindungsmittels und Verschieben der Biomatrixschichten aufeinander durch das Wundbehandlungsmaterial durchgehende Perforationen erhalten bleiben.

12. Verfahren zur Herstellung eines Wundbehandlungsmaterials gemäß einem der vorhergehenden Ansprüche, umfassend die Schritte
a1) Aufbringen eines Klebers auf eine Biomatrixschicht und Aufbringen einer weiteren Biomatrixschicht auf der mit Kleber beschichteten ersten Biomatrixschicht, gegebenenfalls Aufbringen weiterer Kleber- und Biomatrixschichten bis zum Erhalt der gewünschten Gesamtdicke des Wundbehandlungsmaterials und Perforation der miteinander verbundenen Biomatrixschichten;
oder
a2) Perforation der Biomatrixschichten, Aufbringen eines Klebers auf eine perforierte Biomatrixschicht und Aufbringen einer weiteren perforierten Biomatrixschicht auf der mit Kleber beschichteten ersten Biomatrixschicht, gegebenenfalls Aufbringen weiterer Kleber- und Biomatrixschichten bis zum Erhalt der gewünschten Gesamtdicke des Wundbehandlungsmaterials, wobei die perforierten Biomatrixschichten so angeordnet werden, dass bei Auflösung des Klebers durch die Gesamtdicke des Wundbehandlungsmaterials durchgehende Perforationen gebildet werden.
b) gegebenenfalls Trocknen des Klebers; sowie
c) gegebenenfalls Sterilisieren und/oder Konfektionieren.

13. Wundbehandlungsmaterial nach einem der Ansprüche 1 bis 11, welches außerdem eine oder mehrere Deckschichten synthetischer oder semi-synthetischer Wundbehandlungsmaterialien aufweist.

14. Wundbehandlungsmaterial nach einem der Ansprüche 1 bis 11 oder 13 zur Verwendung als Mittel zur Blutstillung sowie in der Behandlung von akuten, chronischen und exsudierenden Wunden, zur Verwendung als Implantat sowie zur Verwendung in der Vakuum-unterstützten Wundbehandlungstherapie.

15. Wündbehandlungsmittel umfassend ein Wundbehandlungsmaterial nach einem der Ansprüche 1 bis 11 oder 13 bis 14, sowie eine vakuumfeste Abdeckungsfolie.
